# FASCICULE DE BREVET EUROPEEN

(11) **EP 0 690 069 B1**
(45) Date de publication et mention de la délivrance du brevet: **26.01.2000**
(21) Numéro de dépôt: 95401544.2
(22) Date de dépôt: 28.06.1995
(51) Int. Cl.: C07J 5/00, C07J 17/00, C07J 31/00

(54) **Nouveau procédé de préparation de dérivés stéroides 20-oxo 17alpha,21-dihydroxylés et nouveaux intermédiaires**
Verfahren zur Herstellung von 20-oxo, 17-alpha, 21-dihydroxy Steroidederivate und neue Zwischenprodukte dafür
Process for the preparation of 20-oxo, 17-alpha, 21 dihydroxy steroid derivatives and new intermediates thereof

(30) Priorité: 01.07.1994 FR 9408140
(43) Date de publication de la demande: 03.01.1996
(73) Titulaire: HOECHST MARION ROUSSEL, 92800 Puteaux (FR)
(72) Inventeur: Lett, Robert, F-75020 Paris (FR); Melnyk, Oleg, F-59370 Mons en Baroeul (FR)
(74) Mandataire: Vieillefosse, Jean-Claude

(56) Documents cités:
- EP-A- 0 531 212
- HETEROCYCLES, vol. 18, 1982 JP, pages 83-86, D. L. FLYNN ET AL 'Chemistry of 2,2-dimethyl-1,3-dioxole. Two-carbon homologation of carbonyl compounds to alpha-ketoaldeydes and dihydroxyacetonyl moities.'

## Description

L'invention a pour objet un nouveau procédé de préparation de dérivés stéroïdes 20-oxo 17α, 21-dihydroxylés et des nouveaux intermédiaires.

L'invention a ainsi pour objet un procédé de préparation des composés de formule (I) : 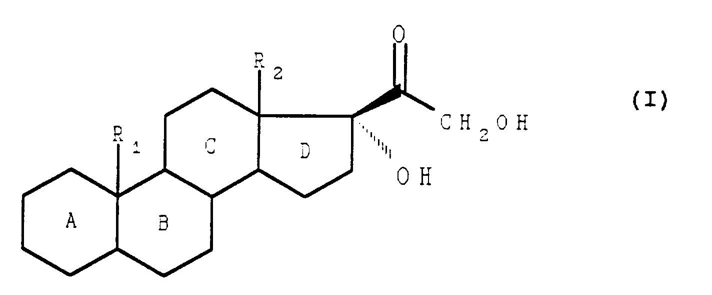 dans laquelle R₁ représente un atome d'hydrogène ou un radical alkyle renfermant de 1 à 4 atomes de carbone, R₂ représente un radical alkyle renfermant de 1 à 4 atomes de carbone, les noyaux A, B, C et D portent une ou plusieurs doubles liaisons et sont éventuellement substitués par une ou plusieurs fonctions hydroxyle ou cétone, le cas échéant protégées, par un ou plusieurs atomes d'halogène ou par un ou plusieurs radicaux alkyle ou alkyloxy renfermant de 1 à 4 atomes de carbone, ou par un ou plusieurs radicaux alkényle ou alkynyle renfermant de 2 à 4 atomes de carbone, caractérisé en ce que l'on traite un composé de formule (II) : 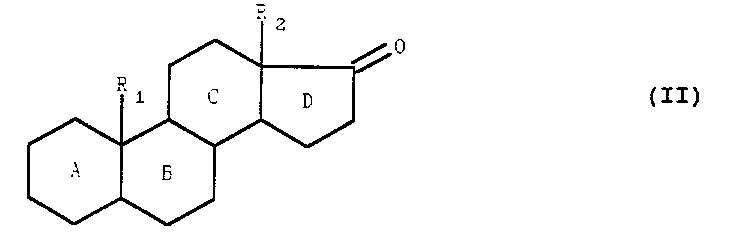 dans laquelle R₁, R₂, A, B, C et D sont définis comme précédemment, par un réactif de formule (R) : 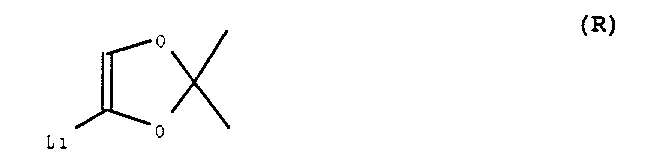 pour obtenir un composé de formule (III) : 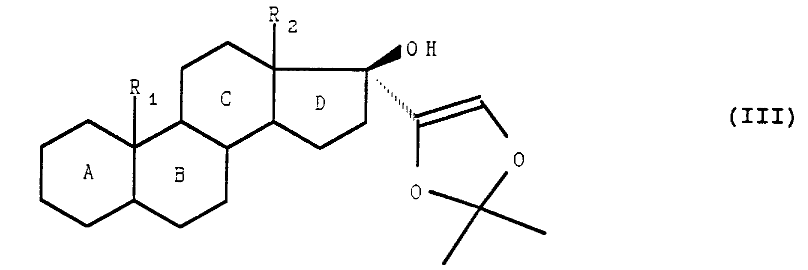 dans laquelle R₁, R₂, A, B, C et D sont définis comme précédemment, que l'on soumet à l'action d'un halogénure d'aryl sulfényle de formule (S) :

Ar-S-Hal (S)

dans laquelle Ar représente un radical phényle éventuellement substitué et Hal représente un atome d'halogène, en présence d'une base, pour obtenir intermédiairement un composé de formule (IV) : 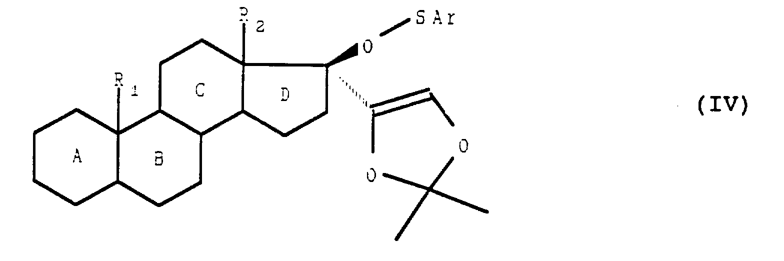 dans laquelle Ar est défini comme précédemment, que l'on réarrange, le cas échéant, en présence d'une base, en sulfoxyde de formule (V) : 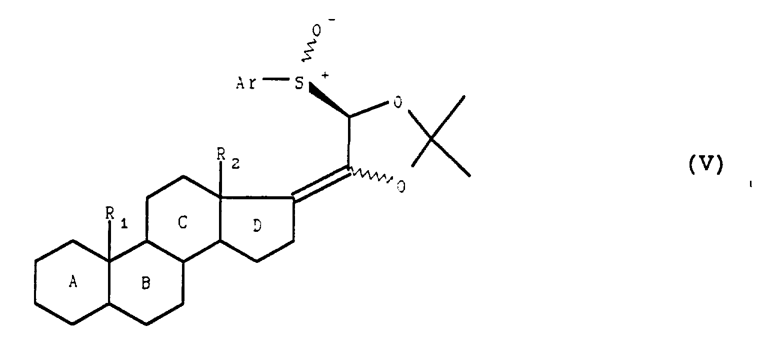 lequel se présente sous la forme d'un mélange de diastéréo-isomères, de configuration E + Z, au niveau de la double liaison 17(20), sulfoxydes que l'on soumet à l'action d'un agent d'épimérisation, pour obtenir les sulfoxydes de formule (VI) : 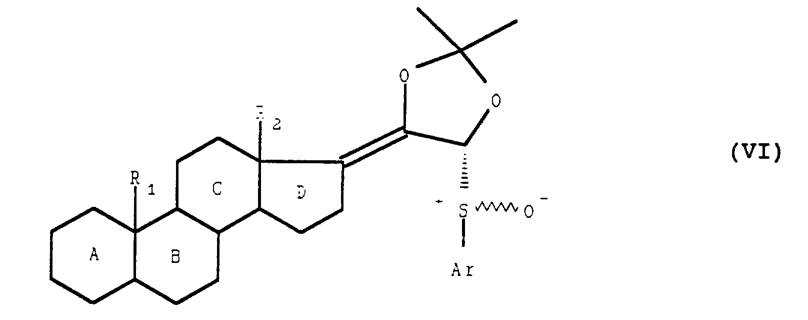 lesquels se présentent très majoritairement sous la forme d'un mélange de diastéréoisomères, de configuration (Z) au niveau de la double liaison 17(20), et qui sont en équilibre avec le sulfénate de formule (VII) : 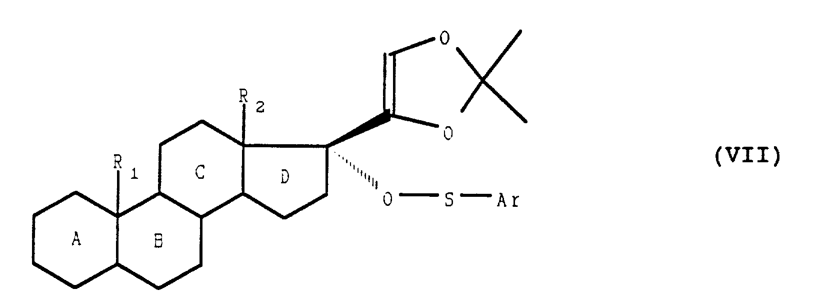 que l'on clive par un thiophile pour obtenir un composé de formule (VIII) : 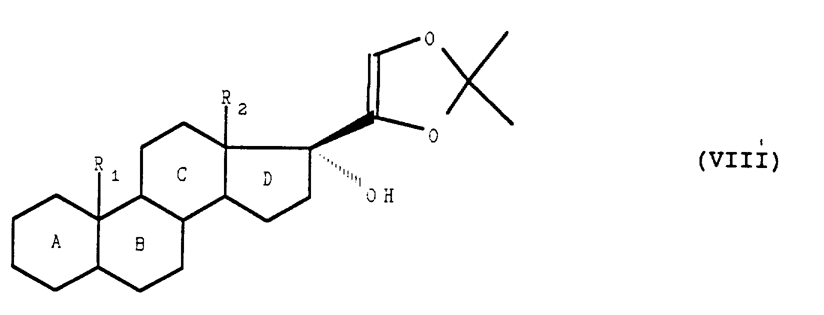 que l'on traite par un agent d'hydrolyse acide pour obtenir le composé de formule (I) attendu.

Lorsque R₁ représente un radical alkyle, il s'agit de préférence du radical méthyle ou éthyle.

R₂ représente de préférence un radical méthyle ou éthyle.

Lorsque les noyaux A, B, C et D portent une ou plusieurs doubles liaisons, il s'agit de préférence de doubles liaisons en 1(2), 3(4), 4(5) ou 9(11) ou d'un système de doubles liaisons conjuguées en 3(4) et 5(6) ou en 4(5) et 6(7) ou en 1(2) et 4(5) ou d'un système aromatique de trois doubles liaisons 1,3,5(10) ou d'un système de trois doubles liaisons 1(2), 4(5), 6(7).

Lorsque les cycles, A, B, C et D sont substitués par une ou plusieurs fonctions hydroxyles, il s'agit de préférence d'une fonction hydroxyle en 3, en 9 ou en 11. Lorsque la ou les fonctions hydroxyles sont protégées, il peut s'agir de tout type de protection connu de l'homme du métier et de préférence d'une protection sous la forme d'esters d'acides organiques, par exemple de l'acide acétique, propionique, formique ou benzoïque, ou d'éthers d'alkyle inférieur, par exemple de méthyle ou d'éthyle, d'alcoxy, alcoxyalkyle, par exemple de méthoxyéthoxyméthyle, d'aralkyle, par exemple de benzyle ou phénéthyle, d'aryle, par exemple de phényle éventuellement substitué, d'éthers silylés, par exemple de trialkylsilyle tel que triméthyl ou diméthyl-tert-butylsilyle, de triarylsilyle tel que triphénylsilyle ou de diarylalkylsilyle tel que diphényl tert-butylsilyle, ou encore d'éther de tétrahydropyranyle.

Lorsque les cycles A, B, C et D sont substitués par une ou plusieurs fonctions cétones, il s'agit de préférence d'une fonction cétone en 3 ou en 11. Lorsque la fonction cétone en 3 est protégée, il s'agit de préférence d'une protection sous la forme d'un cétal ou d'un thiocétal cyclique ou non, ou d'un éther ou ester d'énol ou encore d'une oxime, hydrazone ou semi-carbazone et lorsque la fonction cétone en 11 est protégée, il s'agit de préférence d'une protection sous la forme d'un éther ou ester d'énol. Des exemples de protections de cétones sous forme d'esters et d'éthers sont fournis plus haut.

Lorsque les cycles A, B, C et D sont substitués par un ou plusieurs atomes d'halogène, il s'agit de préférence d'un atome de fluor, de chlore ou de brome, en position 6 ou 9α.

Lorsque les cycles A, B, C et D sont substitués par un ou plusieurs radicaux alkyles, il s'agit de préférence du radical méthyle ou éthyle en 2, 6, 7, en 16α ou en 16β.

Lorsque les cycles A, B, C et D sont substitués par un ou plusieurs radicaux alkyloxy, il s'agit de préférence d'un radical méthoxy ou éthoxy en 3 ou 11β.

Lorsque les cycles A, B, C et D sont substitués par un ou plusieurs radicaux alkényles, il s'agit de préférence du radical vinyle ou allyle en position 6 ou 11β.

Lorsque les cycles A, B, C et D sont substitués par un ou plusieurs radicaux alkynyles, il s'agit de préférence du radical éthynyle en position 6 ou 11β.

L'invention a notamment pour objet un procédé tel que défini précédemment caractérisé en ce que l'on utilise au départ un composé de formule (II) répondant à la formule (II') : 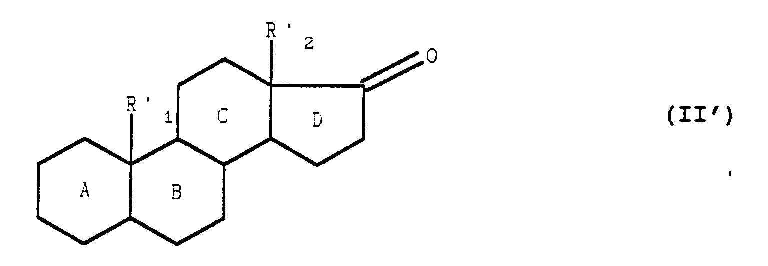 dans laquelle R'₁ représente un atome d'hydrogène ou un radical méthyle, R'₂ représente un radical méthyle ou éthyle, les noyaux A, B, C et D portent une ou plusieurs doubles liaisons en position 1(2), 3(4), 4(5) ou 9(11) ou 3(4) et 5(6) ou 4(5) et 6(7) ou 1(2) et 4(5) ou 1,3,5(10) ou 1(2), 4(5) et 6(7) et sont éventuellement substitués par une ou plusieurs fonctions hydroxyle en 3, 9 et/ou 11, par une ou deux fonctions cétone en 3 et/ou 11, la fonction cétone en 3 étant protégée, par un ou deux atomes de fluor, de chlore ou de brome en 6 et/ou 9α, par un ou plusieurs radicaux méthyle ou éthyle en 2, 6, 7 et/ou 16α ou 16β, par un ou deux radicaux méthoxy ou éthoxy en 3 et/ou 11β, par un radical vinyle ou allyle en 11β ou par un radical éthynyle en 11β.

L'invention a plus particulièrement pour objet un procédé tel que défini précédemment, caractérisé en ce que l'on utilise au départ un composé de formule (II) répondant à la formule (II'') : 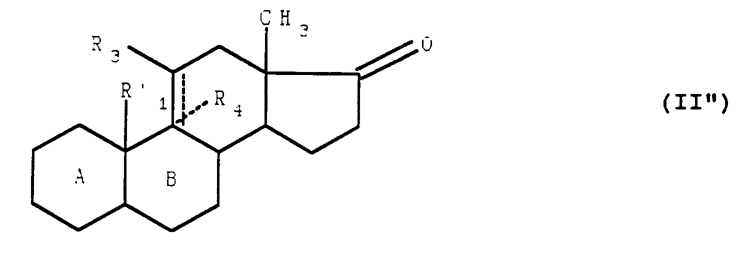 dans laquelle R'₁ est défini comme précédemment, le trait pointillé en 9(11) représente une éventuelle seconde liaison, R₃ représente un atome d'hydrogène, un radical oxo ou un radical hydroxy éventuellement protégé sous forme d'éther ou d'ester, R₄ représente un atome d'hydrogène, un radical hydroxy ou un atome de fluor, les cycles A et B représentent un reste : 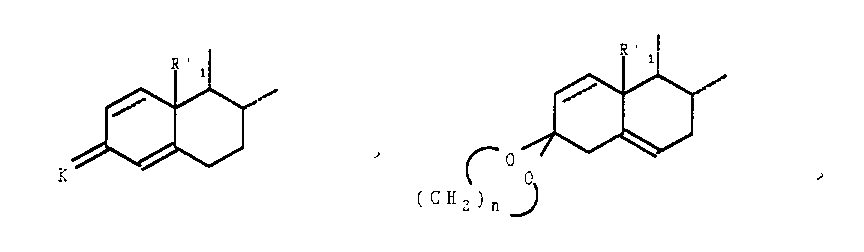 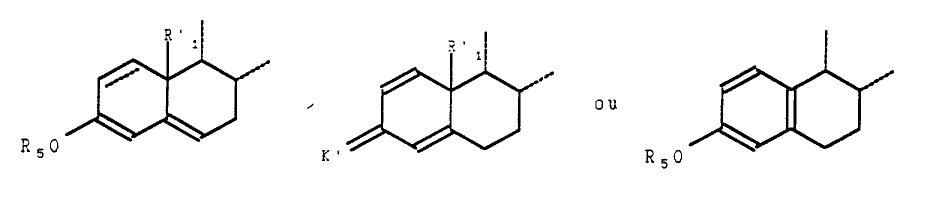 dans lequel le trait pointillé dans le cycle A représente une éventuelle seconde liaison, K représente un radical oxo protégé sous la forme : 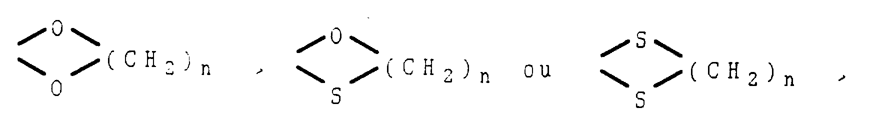 n étant égal à 2 ou 3, R₅ représente un reste éther ou ester, K' représente un radical oxo protégé sous la forme oxime, hydrazone ou semi-carbazone.

Les valeurs de R₅ sont notamment les valeurs préférées des restes éthers et esters mentionnées plus haut.

Il va de soi que la seconde liaison en 9(11) n'est le cas échéant, présente que lorsque les substitutions en 9 et 11 l'autorisent, et que les produits de formule (II), (II') et (II") sont des produits connus ou préparables par des procédés connus de l'homme du métier.

Le réactif R est de préférence préparé in situ et la condensation sur le composé de formule (II) est effectuée de préférence dans un éther tel que le tétrahydrofuranne ou le dioxanne, à une température de l'ordre de -70 à -80°C.

L'agent de sulfénylation de formule (S) est de préférence le chlorure ou bromure de phényl, d'ortho-nitrophényle ou d'ortho-paradinitrophényl sulfényle.

La réaction de sulfénylation est effectuée en présence d'une base qui est notamment une amine tertiaire telle que la triéthylamine, la pyridine, la diméthylaminopyridine, le N-méthylimidazole ou le diazabicyclo-octane, ou encore un mélange de ces bases. On opère de préférence dans les mêmes conditions de solvant que ci-dessus et à une température se situant de préférence entre -80 et -40°C.

Le réarrangement en sulfoxyde de formule (V) est effectué thermiquement en laissant remonter la température jusqu'à la température ambiante. On ajoute de préférence au milieu réactionnel un excès de base qui est notamment la triéthylamine, la pyridine ou le diazabicyclo-octane, si l'on opère en présence de diméthylaminopyridine ou de N-méthylimidazole.

Si désiré, le mélange de diastéréoisomères obtenu peut être séparé, par exemple par chromatographie.

L'agent d'épimérisation que l'on fait agir sur le mélange de sulfoxydes de formule (V) est une base forte, par exemple un hydroxyde d'ammonium tel que l'hydroxyde de tétrabutyl ou de N-benzyltriméthylammonium ou un hydroxyde alcalin, notamment la soude, la potasse ou la lithine, ou encore une autre base azotée. On peut encore utiliser un carbonate ou bicarbonate alcalin. On opère en milieu polaire, aqueux ou alcoolique, par exemple méthanolique et de préférence en présence d'un cosolvant qui peut être notamment le diméthylsulfoxyde ou le diméthylformamide.

Le clivage du sulfénate de formule (VII) est effectué par action d'un agent thiophile qui est de préférence l'une des bases fortes mentionnées plus haut pour l'épimérisation, laquelle déplace ainsi l'équilibre dans ladite épimérisation. Toutefois, tout agent thiophile classique tel qu'un trialkylphosphite comme le triméthyl ou triéthylphosphite, une amine secondaire telle que la diéthylamine ou la pipéridine ou encore un thiolate tel que ArS^{⊖}, par exemple le thiophénolate de sodium ou le thiophénol en présence d'une amine tertiaire.

L'hydrolyse du composé de formule (VIII) est réalisée par action d'un acide minéral ou organique, par exemple l'acide chlorhydrique, bromhydrique, sulfonique, formique, acétique ou paratoluène sulfonique, l'acide chlorhydrique étant préféré. Elle peut être effectuée en milieu homogène, mais on opère avantageusement en milieu biphasique, en présence d'eau et d'un cosolvant organique halogéné tel que le chlorure de méthylène, d'un ester tel que l'acétate d'éthyle, d'un éther tel que l'éther éthylique ou encore d'un solvant aromatique tel que le benzène ou le toluène.

L'invention a notamment pour objet un procédé tel que défini précédemment, caractérisé en ce que l'on opère sans isoler intermédiairement les composés de formules (III) à (VII).

Les conditions opératoires sont celles qui ont été décrites plus haut. Un schéma du procédé dans son ensemble figure ci-après.

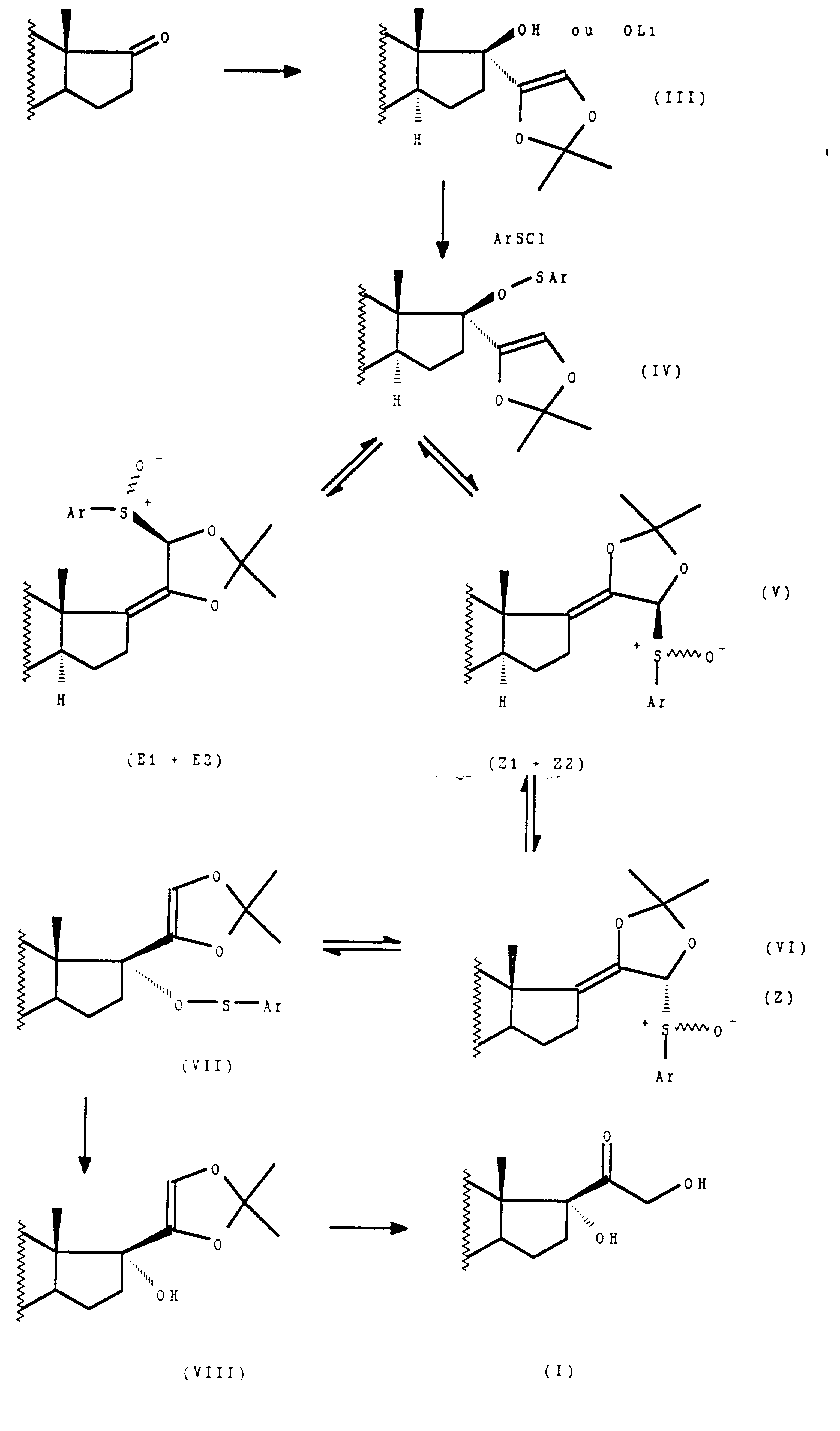

Les corticostéroïdes de formule (I) sont connus le plus souvent de longue date, qu'il s'agisse des composés dans lesquelles les éventuelles fonctions réactives sont protégées comme prévu dans la présente demande ou non, ou peuvent être utilisés pour préparer des produits connus, par des procédés eux-mêmes connus soit de simples déprotections des fonctions protégées, soit de transformations chimiques plus élaborées.

L'hydrolyse d'un composé de type (III) obtenu par action du réactif (R) sur la méthylestrone a déjà été tentée par Mitscher et coll. Heterocycles 1982, 18, 83-86, dans le but d'obtenir la 17-dihydroxycétone correspondante, c'est-à-dire de configuration inverse des composés de formule (I) de l'invention. Cette hydrolyse n'a pu être réalisée, les auteurs n'ayant obtenu que le mélange d'énol-aldéhydes E et Z de formule : 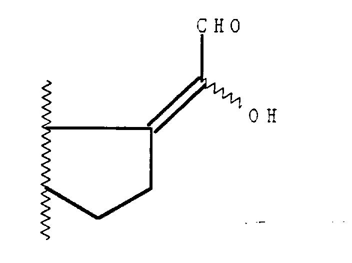 résultant de l'élimination du 17β-OH.

Au niveau de l'hydrolyse finale du composé de formule (VIII), l'invention a donc résolu ce problème.

D'autres exemples d'accès à des structures de type cortisonique via des composés dérivés du 1,4-dioxène ou du 1,2-diméthoxyéthylène (brevets allemands 2521231, 2603266, 2655104 et US 4089852, Tet. Letters 1985, 26, 4925-4928) ont été décrits. Ceux-ci ne font toutefois pas appel à des réarrangements tels que ceux décrits dans la présente demande. Des réarrangements de sulfénates allyliques en sulfoxydes allyliques ont été décrits par des synthèses de corticostéroïdes notamment dans le brevet allemand 2256866 ou les références J. Org. Chem. 1976, 41, 2312-2314 ou 1979, 44, 1582-1584. Ils indiquent toutefois des intermédiaires de nature complètement différente.

Les composés de formules (IV), (V), (VI), (VII) et (VIII), ainsi que les composés de formule (III) à l'exception du dérivé de la méthylestrone, sont nouveaux et l'invention a encore pour objet ces composés à titre de produits industriels nouveaux et notamment à titre d'intermédiaires nécessaires à la mise en oeuvre du procédé de l'invention.

Les exemples suivants illustrent l'invention, sans toutefois la limiter.

### EXEMPLE 1 : 17α,21-dihydroxy 3-méthoxy 19-nor pregna 1,3,5(10)trièn-20-one

### STADE A : 20,21-(1-méthyléthylidène acétal cyclique) de (17alpha) 3-méthoxy 19-norpregna 1,3,5(10),20-tétraène-17,20,21-triol

2,43 ml (3,89 mmoles) de n-butyllithium 1,6 M dans le n-hexane sont additionnés en 5 minutes à 478 µl (4,05 mmoles) de vinylène acétonide en solution dans 5 ml de tétrahydrofuranne anhydre à -78°C et sous argon. Après 15 minutes, la réaction est placée dans un bain à -30°C et agitée 1 h. 552 mg (1,94 mmole) de méthyloestrone en solution dans 13 ml de tétrahydrofuranne anhydre sont alors ajoutés en 20 minutes. Après 1 h, le milieu est hydrolysé avec 10 ml d'une solution aqueuse saturée en carbonate acide de sodium, puis extrait avec 3 x 50 ml d'éther. Les phases organiques réunies sont lavées avec 50 ml d'une solution aqueuse saturée en carbonate acide de sodium puis filtrées sur sulfate de sodium. Après évaporation des solvants, le produit brut est purifié par chromatographie sur silice (CH₂Cl₂98/AcOEt 2) pour donner 630 mg (84 %) de produit attendu.
RMN ¹H 200 MHz (C₅D₅N) :
7,10 (d, 1H, H₁, J₁₋₂ = 8,5 Hz) ; 6,68 (dd, 1H, H₂, J₂₋₁ = 8,5 Hz, J₂₋₄ = 3 Hz) ; 6,57 (d, 1H, H₄, J₄₋₂ = 3 Hz) ; 6,35 (s, 1H, H₂₁) ; 6,30 (sl, 1H éch., OH) ; 3,49 (s, 3H, OMe) ; 1,39 (s, 3H, C(CH₃)) ; 1,36 (s, 3H, C(CH₃)) ; 0,98 (s, 3H, Me₁₈).

| IR (CHCl₃) : | |
|---|---|
| 3575 | (OH libre) |
| 3490 | (OH ass.) |
| 1609-1576-1500 | (Ar) |
| 1383 et 1373 | (C(CH₃)₂). |

### STADE B : 1-méthyléthylidène acétal cyclique de 3-méthoxy 21-(phénylsulfinyl) 19-norpregna 1,3,5(10),17(20)-tétraène-20,21-diol

85,6 mg (0,22 mmole) de produit obtenu au stade A, 4,0 mg (33 µmoles) de diméthylaminopyridine et 155 µl (1,11 mmole) de triéthylamine sont solubilisés à -40°C et sous argon dans 5 ml de tétrahydrofuranne anhydre, 25 µl (0,21 mmole)) de chlorure de benzènesulfényle sont ajoutés en 30 minutes. 5 µl (43 µmoles) de chlorure de benzènesulfényle sont encore ajoutés et l'agitation poursuivie pendant 1 h 30. 250 µl (1,79 mmole) de triéthylamine sont additionnés et le bain froid enlevé. Le milieu est hydrolysé à température ambiante avec 40 ml d'une solution aqueuse saturée en carbonate acide de sodium, 40 ml d'eau puis extrait avec 3 x 50 ml de dichlorométhane. Les phases organiques réunies sont filtrées sur sulfate de sodium après avoir ajouté 5 gouttes de pyridine. Après évaporation des solvants, le produit brut est purifié par chromatographie sur silice (CH₂Cl₂ 95/AcOEt 5/pyridine 0,2) pour donner 82,1 mg (75 %) de sulfoxydes attendus. Après chromatographie sur silice (éluant CH₂Cl₂ 96/AcOEt 4/pyridine 0,2) on obtient 14 % de produit sous forme d'isomère Z et 27 % de produit attendu sous forme d'isomère E).
Isomère E : mélange de 2 diastéréoisomères (8/2) (21R, épimères au soufre)
RMN ¹H 200 MHz (C₅D₅N) :
7,8-7,2 (m, 5H, SPh) ; 7,11 (d, 1H, H₁, J₁₋₂ = 8,5 Hz) ; 6,73 (dd, 1H, H₂, J₂₋₁ = 8,5 Hz, J₂₋₄ = 3 Hz) ; 6,61 (d, 1H, H₄, J₄₋₂ = 3 Hz) ; 5,76 (s, 0,8 H, H_{21α}) ; 5,58 (s, 0,2 H, H_{21α}) ; 3,58 (s, 2,4 H, OMe) ; 3,50 (s, 0,6 H, OMe) ; 1,63 (s, 2,4 H, C(CH₃)) ; 1,22 (s, 0,6 H, C(CH₃)) ; 1,18 (s, 2,4 H, C(CH₃)) ; 1,15 (s, 0,6 H, C(CH₃)) ; 0,89 (s, 0,6 H, Me₁₈) ; 1,30 (s, 2,4 H, Me₁₈).
Isomère Z : mélange de 2 diastéréoisomères (8/2) (21S, épimères au soufre)
RMN ¹H 200 MHz (C₅D₅N) :
7,8-7,2 (m, 5H, SPh) ; 7,09 (d, 1H, H₁, J₁₋₂ = 8,5 Hz) ; 6,69
(dd, 1H, H₂, J₂₋₁ = 8,5 Hz, J₂₋₄ = 3 Hz) ; 6,60 (d, 1H, H₄, J₄₋₂ = 3 Hz) ; 5,41 (s, 0,8 H, H_{21α}) ; 5,36 (s, 0,2 H, H_{21α}) ; 3,49 (s, 3 H, OMe) ; 1,56 (s, 2,4 H, C(CH₃)) ; 1,21 (s, 0,6 H, C(CH₃)) ; 1,18 (s, 2,4 H, C(CH₃)) ; 1,15 (s, 0,6 H, C(CH₃)) ; 0,82 (s, 0,6 H, Me₁₈) ; 0,75 (s, 2,4 H, Me₁₈).

### STADE C : 20,21-(1-méthyléthylidène acétal cyclique) de 3-méthoxy 19-norpregna 1,3,5(10),20-tétraène-17,20,21-triol

30,4 mg (61,7 µmoles) de sulfoxydes E obtenus au stade B et 0,5 ml (1,1 mmole, 40 % dans le méthanol) de triton B (N-benzyl triméthylammonium hydroxyde) sont agités 16 heures à température ambiante dans 2 ml de diméthylsulfoxyde. Le milieu est ensuite hydrolysé avec 20 ml d'une solution aqueuse saturée par du carbonate acide de sodium puis extrait avec 100 ml d'un mélange CH₂Cl₂ 1/AcOEt 1. La phase organique est lavée avec 2 x 25 ml d'une solution aqueuse saturée par du carbonate acide de sodium diluée 5 fois puis filtrée sur une courte colonne de sulfate de sodium après avoir ajouté 1 goutte de pyridine. Après évaporation des solvants, le produit brut est purifié par chromatographie sur silice (toluène 96,2/AcOEt 3,6/pyridine 0,2) pour donner 9,3 mg (39 %) de produit attendu.

16,2 mg (32,9 µmoles) de sulfoxydes Z obtenus au stade B et 0,25 ml (0,55 mmole, 40 % dans le méthanol) de triton B sont agités 16 heures à température ambiante dans 2 ml de diméthylsulfoxyde. Le traitement et la purification sont identiques au mode opératoire précédent. On obtient 8,2 mg (65 %) de produit attendu.
F = 121-122°C (Kofler)
RMN ¹H 200 MHz (C₅D₅N) :
7,09 (d, 1H, H₁, J₁₋₂ = 8,5 Hz) ; 6,68 (dd, 1H, H₂, J₂₋₁ = 8,5 Hz, J₂₋₄ = 3 Hz) ; 6,58 (d, 1H, H₄, J₄₋₂ = 3 Hz) ; 6,30 (s, 1 H, H₂₁) ; 6,02 (sl, 1 H éch., OH) ; 3,50 (s, 3 H, OMe) ; 1,38 (s, 3 H, C(CH₃)) ; 1,28 (s, 3H, C(CH₃) ; 0,71 (s, 3 H, Me₁₈).
SM (SIMS matrice BNA) : m/z = 384 (M⁺) , 367 (MH⁺-H₂₀) ; 326 (M⁺ - Me₂CO).
IR (CHCl₃) : 3586 (OH) ; 1608-1575-1500 (MeO-Ar) ; 1383 et 1373 (C(CH₃)₂).
UV (EtOH) 278 (2400) ; 287 (2300).

### STADE D : 17α,21-dihydroxy 3-méthoxy 19-norpregna 1,3,5(10)-trièn 20-one

0,33 ml (0,33 mmole) d'acide chlorhydrique 1N saturé par du chlorure de sodium est ajouté à 14,4 mg (37,4 µmoles) de produit obtenu au stade C, en solution dans 3,3 ml de dichlorométhane à température ambiante. Le milieu est agité fortement 15 minutes puis traité et purifié comme précédemment pour donner 5,9 mg (46 %) de produit attendu.
F = 182°C (Kofler)
RMN ¹H 200 MHz (CDCl₃) :
7,20 (d, 1H, H₁, J₁₋₂ = 8,5 Hz) ; 6,72 (dd, 1H, H₂, J₂₋₁ = 8,5 Hz, J₂₋₄ = 3 Hz) ; 6,64 (d, 1H, H₄, J₄₋₂ = 3 Hz) ; 4,71 (dd, 1 H, H₂₁, ²j = 20 Hz, J_{21-OH} = 5 Hz) ; 4,35 (dd, 1 H, H₂₁, ²J = 20 Hz, J_{21-OH} = 5 Hz) ; 3,78 (s, 3 H, OMe) ; 3,12 (t, 1H éch., CH₂OH, J_{OH-21} = 5 Hz) ; 2,19 (s, 1H éch., OH) ; 0,71 (s, 3H, Me₁₈).
SM (FD matrice BNA) : m/z = 344 (M⁺, 100 %).
IR (CHCl₃) : 3610 (OH libre) ; 3505 (OH ass.) ; 1700 (C=O) ; 1575 et 1500 (MeO-Ar).
UV (EtOH) 279 (2100) ; 287 (2000).

### EXEMPLE 2 : 17α,21-dihydroxy 3-méthoxy 19-nor pregna 1,3,5(10)trièn-20-one

### STADE A : 3-méthoxy 20,21-(diméthylméthylène dioxy) 17β-pregna 1,3,5(10) ,20-tétraèn-17α-ol

550 µl (0,88 mmole) de n-butyllithium 1,6 M dans le n-hexane sont additionnés en 10 minutes à 105,3 µl (0,89 mmole) de vinylène acétonide en solution dans 1 ml de tétrahydrofuranne anhydre à -78°C et sous argon. Le ballon est alors placé dans un bain d'acétone à -40°C pendant 1 h. 110,2 mg (0,387 mmole) de méthyloestrone en solution dans 4,5 ml de tétrahydrofuranne sont ensuite ajoutés en 10 minutes. Après 1 h, 70,8 mg (0,58 mmole) de diméthylaminopyridine en solution dans 1 ml de tétrahydrofuranne sont ajoutés. 200 µl (1,7 mmole) de chlorure de benzène sulfényle sont alors additionnés en 2 h 15. Dès l'addition terminée, l'excès de chlorure de benzène sulfényle est détruit avec 100 µl (2,48 mmoles) de MeOH. 436 mg (3,89 mmoles) de DABCO sont ajoutés puis le bain froid enlevé. Le milieu est dilué à température ambiante avec 10 ml de diméthylsulfoxyde et chauffé à 50°C sous un vide partiel de 30 mmHg (début du chauffage 30 minutes après avoir enlevé le bain d'acétone). Après 45 minutes, 1,5 ml (3,8 mmoles, 40 % dans l'eau) de triton B est ajouté à température ambiante et sous argon. Le milieu est agité 1 h 15 puis refroidi à 0°C pour être hydrolysé avec 100 ml d'une solution aqueuse saturée par du carbonate acide de sodium. La phase aqueuse est extraite avec 6 x 50 ml de cyclohexane. Les phases organiques réunies sont lavées avec 50 ml d'une solution aqueuse saturée par du carbonate acide de sodium puis filtrées sur une courte colonne de sulfate de sodium après avoir ajouté 0,5 ml de pyridine. Après évaporation des solvants, le produit brut est purifié par chromatographie flash sur silice (toluène 96,4/AcOEt 3,6/pyridine 0,2) pour donner 66,1 mg ( 44 %) de 3-méthoxy 20,21-(diméthylméthylènedioxy) 17β-pregna 1,3,5(10) ,20-tétraèn-17α-ol.

### STADE B : 17α,21-dihydroxy 3-méthoxy 19-nor pregna 1,3,5(10)trièn-20-one

On opère comme indiqué au stade D de l'exemple 1 et obtient le produit attendu.

### EXEMPLE 3 : 17α,21-dihydroxy pregn-4-ène 3,11,20-trione

### STADE A : (17alpha) 17-hydroxy 3-méthoxy 20,21-[(1-méthyléthylidène) bis(oxy)] pregna-3,5,20-trîèn-11-one

1,20 ml (1,92 mmole) de n-butyllithium 1,6 M dans l'hexane est additionné en 5 minutes à 237 µl (1,98 mmole) de vinylène acétonide en solution dans 5 ml de tétrahydrofuranne à -78°C et sous argon. Après 15 minutes, le ballon est placé dans un bain d'acétone à -40°C pendant 1 heure. Le milieu est ensuite refroidi à -78°C. 263,8 mg (0,84 mmole) de 3-méthoxy androsta 3,5-diène-11,17-dione en solution dans 5 ml de tétrahydrofuranne sont ajoutés en 15 minutes. Après 45 minutes, le milieu est hydrolysé avec 30 ml d'une solution aqueuse saturée par du carbonate acide de sodium puis extrait avec 2 x 100 ml d'éther. Les phases organiques réunies sont lavées avec 2 x 30 ml d'une solution aqueuse saturée par du carbonate acide de sodium puis filtrées sur une courte colonne de sulfate de sodium après avoir ajouté 5 gouttes de pyridine. Après évaporation des solvants, le produit brut est purifié par chromatographie flash sur silice (toluène 87/AcOEt 13/pyridine 0,2) pour donner une solution de produit attendu pur à laquelle 102,6 mg (0,84 mmole) de diméthylaminopyridine sont ajoutés avant l'évaporation des solvants. On obtient 350,0 mg d'un mélange contenant 71 % en masse de produit attendu soit 247,4 mg de produit pur.
RMN ¹H 200 MHz (CDCl₃ + 0,4 % de C₅D₅N) :
6,12 (s, 1H, H₂₁) ; 5,19 (m, 1H, H₆) ; 5,09 (d, 1H, H₄, J = 1Hz) ; 3,57 (s, 3H, OMe) ; 1,56 (s, 3H, C(CH₃)) ; 1,50 (s, 3H, C(CH₃)) ; 1,16 (s, 3H, Me₁₉) ; 1,07 (s, 3H, Me₁₈).

### STADE B : 3-méthoxy 20,21-[(1-méthyléthylidène) bis(oxy)] 21-(phénylsulfinyl) pregna-3,5,17(20)-trièn-11-one

92,4 mg (0,22 mmole) de produit obtenu au stade A stabilisé avec 70,2 µl (0,88 mmole) de N-méthyl imidazole et 155 µl (1,10 mmole) de triéthylamine sont solubilisés dans 2,4 ml de tétrahydrofuranne anhydre à -40°C et sous argon. 700 µl (0,54 mmole) de chlorure de benzène sulfényle 0,78 M dans le tétrahydrofuranne sont ajoutés en 1 h 30. Le milieu est hydrolysé à -40°C avec 30 ml d'une solution aqueuse saturée par du carbonate acide de sodium et extrait avec 100 ml puis 50 ml d'éther. Les phases organiques réunies sont lavées avec 3 x 30 ml d'une solution aqueuse saturée par du carbonate acide de sodium puis filtrées sur une courte colonne de sulfate de sodium après avoir ajouté 0,5 ml de pyridine. Après évaporation des solvants, le produit brut est purifié par chromatographie flash sur silice (CH₂Cl₂ 95/AcOEt 5/pyridine 0,2) pour donner 20,0 mg (17 %) d'isomère E et 59,9 mg (51 %) d'isomère Z.
Isomère E : (21R)
RMN ¹H 200 MHz (C₅D₅N) :
7,7-7,1 (m, 5H, Ph) ; 5,72 (s, 1H, H_{21α}) ; 5,09 (m, 2H, H₆, H₄) ; 3,34 (s, 3H, OMe) ; 2,91 (d, 1H, H₁₂, ²J = 12 Hz) ; 2,67 (dd, 1H, Hₓ, J = 12 et 5 Hz) ; 2,48 (d, 1H, H₁₂, ²J = 12 Hz) ; 1,59 (s, 3H, Me₁₉) ; 1,16 (s, 6H, C(CH₃)₂) ; 0,95 (s, 3H, Me₁₈).
Isomères Z : (21S', épimères au soufre)
Isomère Z₁ :
RMN ¹H 200 MHz (C₅D₅N) :
7,7-7,2 (m, 5H, Ph) ; 5,44 (s, 1H, H_{21α}) ; 5,10 (m, 2H, H₆, H₄) ; 3,30 (s, 3H, OMe) ; 3,06 (d, 1H, H_{12β}, ²J = 13 Hz) ; 2,68 (dd, 1H, Hₓ, J = 13 et 4 Hz) ; 2,32 (dl, 1H, H_{12α}, ²J = 13 Hz) ; 1,48 (s, 3H, Me₁₉) ; 1,11 (s, 3H, C(CH₃)) ; 1,09 (s, 3H, C(CH₃) ; 0,75 (s, 3H, Me₁₈).
Isomère Z₂ :
RMN ¹H 200 MHz (C₅D₅N) :
7,75-7,20 (m, 5H, Ph) ; 5,33 (s, 1H, H_{21α} ) ; 5,09 (m, 2H, H₆, H₄) ; 3,29 (s, 3H, OMe) ; 3,07 (d, 1H, H_{12β}, ²J = 13 Hz) ; 2,88 (dd, 1H, Hₓ, J = 17 et 9 Hz) ; 2,68 (dd, 1H, Hₓ, J = 12,5 et 4 Hz) ; 2,32 (dl, 1H, H_{12α}, ²J = 13 Hz) ; 1,17 (s, 3H, Me₁₉) ; 1,12 (s, 3H, C(CH₃)) ; 1,06 (s, 3H, C(CH₃) ; 0,82 (s, 3H, Me₁₈).

### STADE C : (17alpha) 17-hydroxy 3-méthoxy 20,21-[(1-méthyléthylidène) bis(oxy)] pregna-3,5,20-trièn-11-one

### 1ère méthode :

77,3 mg (0,148 mmole) de sulfoxydes obtenus au stade B sont solubilisés à température ambiante et sous argon dans 12 ml de diméthylsulfoxyde. 1,5 ml (3,8 mmoles) de triton B à 40 % dans l'eau sont ajoutés en une fois. Après 15 h d'agitation, le milieu est dilué avec 90 ml d'une solution aqueuse saturée par du carbonate acide de sodium puis extrait avec 2 x 100 ml et 50 ml d'éther. Les phases organiques réunies sont lavées avec 2 x 30 ml d'une solution aqueuse saturée par du carbonate acide de sodium puis filtrées sur une courte colonne de sulfate de sodium, après avoir ajouté 5 gouttes de pyridine. Après l'évaporation des solvants, le produit brut est purifié par chromatographie flash sur silice (CH₂Cl₂ 95/AcOEt 5/pyridine 0,2) pour donner 27,7 mg (45 %) d'alcool 17α attendu.

### 2ème méthode

30,4 mg (58,2 µmoles) de sulfoxydes obtenus au stade B sont solubilisés à température ambiante et sous argon dans 4 ml de pyridine. 0,5 ml (1,26 mmole) de triton B à 40 % dans l'eau sont ajoutés en une fois. Après 15 h d'agitation, le milieu est dilué avec 30 ml d'une solution aqueuse saturée par du carbonate acide de sodium et extrait avec 100 ml puis 50 ml d'éther. Les phases organiques réunies sont lavées avec 2 x 20 ml d'une solution aqueuse saturée par du carbonate acide de sodium puis filtrées sur une courte colonne de sulfate de sodium après avoir ajouté 5 gouttes de pyridine. Après l'évaporation des solvants, le produit brut est purifié par chromatographie flash sur silice (CH₂Cl₂ 95/AcOEt 5/pyridine 0,2) pour donner 13,6 mg (56 %) d'alcool 17α attendu.

### STADE D : 17α,21-dihydroxy pregn-4-ène 3,11,20-trione

0,44 ml (0,44 mmole) d'acide chlorhydrique 1N saturé par du chlorure de sodium est ajouté à 23,4 mg (56,5 µmoles) de produit obtenu au stade C en solution dans 8,75 ml de CH₂Cl₂ à température ambiante et sous argon. Le milieu est agité fortement 4 h 15 puis traité comme précédemment. Le produit brut est purifié par chromatographie sur silice (CH₂Cl₂ 80/Me₂CO 20) pour donner 11,0 mg (54 %) de cortisone 1.
RMN ¹H 300 MHz (CDCl₃) :
6,13 (sl, 1H éch., OH) ; 5,73 (sl, 1H, H₄) ; 4,71 (d, 1H, H₂₁, ²J = 20 Hz) ; 4,29 (d, 1H, H₂₁, ²J = 20 Hz) ; 2,97 (d, 1H, H₁₂, ²J = 12,5 Hz) ; 2,16 (d, 1H, H₁₂, ²J = 12,5 Hz) ; 1,41 (s, 3H, Me₁₉) ; 0,65 (s, 3H, Me₁₈).
SM (EI) : m/z = 360 (M⁺) ; 342 (M⁺ - H₂O) ; 301, 258, 161, 121, 105, 91 (100 %).
IR (CHCl₃) : 3490 (OH) ; 1705 (C=O en 11 et 20) ; 1656 (C=O en 3) ; 1613 (C=C).
UV (EtOH) : 237 (15500).

### EXEMPLE 4 : 17α,21-dihydroxy pregn-4-ène 3,11,20-trione

### STADE A : 3-méthoxy 17β-[20,21-(diméthylméthylènedioxy)] 11-oxo -pregna-3,5,20-trièn-17α-ol

3,10 ml (4,96 mmoles) de n-butyllithium 1,6 M dans l'hexane sont additionnés en 5 minutes à 590 µl (4,98 mmoles) de vinylène acétonide en solution dans 5 ml de tétrahydrofu-ranne à -78°C et sous argon. Après 15 minutes, le ballon est placé dans un bain d'acétone à -40°C pendant 1 heure. Le milieu est ensuite refroidi à -78°C et 654 mg (2,08 mmoles) de 3-méthoxy androsta 3,5-diène 11,17-dione, en solution dans 4 ml de tétrahydrofuranne sont ajoutés en 10 minutes. Après 45 minutes, le milieu est hydrolysé -78°C avec 50 ml d'une solution aqueuse saturée par du carbonate acide de sodium puis extrait avec 3 x 100 ml d'éther. Les phases organiques réunies sont lavées avec 30 ml d'une solution aqueuse saturée 5 par du carbonate acide de sodium puis filtrées sur une courte colonne de sulfate de sodium après avoir ajouté 5 gouttes de pyridine. Après évaporation des solvants, le produit brut est purifié par chromatographie flash sur silice (toluène 87/AcOEt 13/pyridine 0,2) pour donner une solution de (17alpha) 17-hydroxy 3-méthoxy 20,21-[(1-méthyléthylidène) bis(oxy)] pregna-3,5,20-trièn-11-one pur à laquelle on ajoute 830 µl (10,4 mmoles) de N-méthylimidazole. L'évaporation des solvants fournit une huile utilisée rapidement dans l'étape suivante de sulfénylation.

L'huile précédente et 1,46 ml (10,5 mmoles) de triéthylamine sont solubilisés dans 22 ml de tétrahydrofuranne anhydre et 5 ml de dichlorométhane anhydre à -40°C et sous argon. 300 µl (2,56 mmoles) de chlorure de benzène sulfényle sont additionnés en 2 H 20. Le milieu est ensuite hydrolysé à -40°C avec 50 ml d'une solution aqueuse saturée par du carbonate acide de sodium puis extrait avec 3 x 100 ml d'éther. Les phases organiques réunies sont lavées avec 3 x 30 ml d'une solution aqueuse saturée par du carbonate acide de sodium, 50 ml d'une solution aqueuse saturée par du chlorure de sodium puis filtrées sur une courte colonne de sulfate de sodium après avoir ajouté 0,5 ml de pyridine. Le produit brut obtenu par évaporation des solvants est agité 1 h 30 à température ambiante et sous argon avec 4,35 ml (11,02 mmoles, 40 % dans l'eau) de triton B dans 30 ml de diméthylsulfoxyde. Le milieu est ensuite refroidi avec un bain de glace et dilué avec 100 ml d'une solution aqueuse saturée par du carbonate acide de sodium. La phase aqueuse est extraite avec 4 x 100 ml d'éther. Les phases organiques réunies sont lavées avec 4 x 30 ml d'une solution aqueuse saturée par du carbonate acide de sodium, 50 ml d'une solution aqueuse saturée par du chlorure de sodium puis filtrées sur une courte colonne de sulfate de sodium après avoir ajouté 5 gouttes de pyridine. Après évaporation des solvants, le produit brut est purifié par chromatographie flash sur silice (CH₂Cl₂ 95/AcOEt 5/pyridine 0,2) pour donner 369 mg de produit attendu.
RMN ¹H 300 MHz (CDCl₃)
6,09 (s, 1H, H₂₁) ; 5,21 (m, 1H, H₆) ; 5,10 (s, 1H, H₄) ; 3,57 (s, 1H, OMe) ; 2,74 (dl, 1H, H_{12α} , ²J = 12,5 Hz) ; 2,37 (d, 1H, H_{12β}, ²J = 12,5 Hz) ; 1,53 (s, 3H, C(CH₃)) ; 1,50 (s, 3H, C(CH₃)) ; 1,17 (s, 3H, Me₁₉) ; 0,72 (s, 3H, Me₁₈).
SM (SIMS matrice NBA) : m/z = 415 (MH⁺) ; 414 (M⁺, 100 %) ; 397 (MH⁺ - H₂O) ; 356 (M⁺ - Me₂CO) ; 397 (397 - Me₂CO) ; 154, 136.
IR (CHCl₃) : 3588 (OH) ; 1703 (C=0 en 11) ; 1655 et 1628 (C=C) ; 1387 et 1374 (C(CH₃)₂).
UV (EtOH) 220 (4500) ; 236 (21400).

### STADE B : 17α,21-dihydroxy pregn-4-ène 3,11,20-trione

En opérant comme indiqué au stade D de l'exemple 3, on obtient le produit attendu.

### EXEMPLE 5 : 17α,21-dihydroxy pregn-4-ène 3,20-dione

### STADE A : 3-méthoxy 20,21-[(1-méthyléthylidène) bis(oxy)] pregna-3,5,20-trièn-(17alpha)-17-ol

1,40 ml (2,1 mmoles) de n-butyllithium 1,5 M dans l'hexane est additionné en 5 minutes à 250 µl (2,09 mmoles) de vinylène acétonide en solution dans 2 ml de tétrahydrofuranne anhydre à -78°C et sous argon. Après 10 minutes, on laisse remonter la température du milieu réactionnel vers -40°C et maintient 1 heure à cette température. On ajoute 265,4 mg (0,88 mmole) de 3-méthoxy androsta 3,5-dièn-17-one en solution dans 2 ml de tétrahydrofuranne en 5 minutes. Le milieu est encore agité 30 minutes puis hydrolysé à -40°C avec 20 ml d'une solution aqueuse saturée par du carbonate acide de sodium. La phase aqueuse est extraite avec 20 ml puis 80 ml d'éther. Les phases organiques réunies sont lavées avec 20 ml d'une solution aqueuse saturée par du carbonate acide de sodium puis filtrées sur une courte colonne de sulfate de sodium après avoir ajouté 5 gouttes de pyridine. Après l'évaporation des solvants, le produit brut est purifié par chromatographie flash sur silice (toluène 94/AcOEt 6/pyridine 0,2). Les solutions de produit attendu pur issues de la chromatographie flash sont stabilisées avec 23,1 mg de
para diméthylamino pyridine avant l'évaporation des solvants. On obtient ainsi 353,3 mg d'un solide blanc cristallisé conservé à -20°C, correspondant à 93 % de produit pur.
RMN ¹H 200 MHz (CDCl₃ + 0,4 % de pyridine-d₅)
Stabilisé para diméthylamino pyridine
6,01 (s, 1H, H₂₁) ; 5,22 (m, 1H, H₆) ; 5,12 (sl, 1H, H₄) ; 3,56 (s, 3H, OMe) ; 1,55 (s, 3H, C(Me)) ; 1,50 (s, 3H, C(Me)) ; 0,96 (s, 3H, Me₁₉) ; 0,91 (s, 3H, Me₁₈).

### STADE B : 3-méthoxy 20,21-[(1-méthyléthylidène) bis(oxy)] 21-(phénylsulfinyl) pregna-3,5,17(20)-triène

224,1 mg (0,56 mmole) d'alcool obtenu au stade A et 223 µl (2,80 mmoles) de N-méthylimidazole sont solubilisés à -40°C dans 6 ml de tétrahydrofuranne anhydre et sous argon. 390 µl (2,80 mmoles) de triéthylamine sont ajoutés en une fois, 80 µl (0,68 mmoles) de chlorure de benzène sulfényle sont additionnés en 3 h. Le milieu est alors hydrolysé avec 20 ml d'une solution aqueuse saturée par du carbonate acide de sodium. La phase aqueuse est extraite avec 3 x 50 ml d'éther. Les phases organiques réunies sont lavées avec 2 x 20 ml d'une solution aqueuse saturée par du carbonate acide de sodium puis filtrées sur une courte colonne de sulfate de sodium après avoir ajouté 5 gouttes de pyridine. Après l'évaporation des solvants, le produit brut est purifié par chromatographie flash sur silice (toluène 90/AcOEt 10/pyridine 0,2) pour donner les sulfoxydes attendus (4-diastéréoisomères) utilisés directement dans l'étape suivante d'épimérisation.
Sulfoxydes E : 2 diastéréoisomères (21R, épimères au soufre)
RMN ¹H 200 MHz (pyridine-d₅) :
5,71 et 5,54 ppm (s, H_{21α}) ; 5,11 (m, 2H, H₄ et H₆) ; 3,31 (s, 3H, OMe).
Sulfoxydes Z : 2 diastéréoisomères (21S, épimères au soufre)
RMN ¹H 200 MHz (pyridine-d₅) :
5,37 et 5,31 ppm (s, H_{21α}) ; 5,12 (m, 2H, H₄ et H₆) ; 3,30 (s, 3H, OMe) ; 1,55 (s, 3H, Me₁₉) ; 1,16 (s, 3H, Me₁₈) ; 0,80 (s, 3H, C(Me)) ; 0,78 (s, 3H, C(Me)).

### STADE C : 3-méthoxy 20,21-[(1-méthyléthylidène) bis(oxy)] pregna-3,5,20-triène-(17alpha)-17-ol

Les sulfoxydes sont dissous à température ambiante et sous argon dans 2 ml de diméthylsulfoxyde. 0,5 ml (1,1 mmole) de triton B à 40 % dans le méthanol est ajouté en une fois. Après 1 h d'agitation, le milieu est dilué avec 20 ml d'une solution aqueuse saturée par du carbonate acide de sodium puis extrait avec 2 x 50 ml d'éther. Les phases organiques réunies sont lavées avec 2 x 20 ml d'une solution aqueuse saturée par du carbonate acide de sodium puis filtrées sur une courte colonne de sulfate de sodium après avoir ajouté 5 gouttes de pyridine. Après l'évaporation des solvants, le produit brut est purifié par chromatographie flash sur silice (cyclohexane 60/iPr₂O 40/pyridine 0,2) pour donner l'alcool 17α attendu.
RMN ¹H 200 MHz (CDCl₃ + 0,4 % pyridine-d₅) :
6,06 (s, 1H, H₂₁) ; 5,22 (m, 1H, H₆) ; 5,13 (sl, 1H, H₄) ; 3,56 (s, 3H, OMe) ; 1,52 (s, 3H, C(Me) ; 1,51 (s, 3H, C(Me)) ; 0,97 (s, 3H, Me₁₉) ; 0,74 (s, 3H, Me₁₈).

### STADE D : (17alpha)17,21-dihydroxy pregn-4-ène-3,20-dione

On opère comme indiqué à l'exemple 3, stade D et obtient le produit attendu.

## Revendications

1. Procédé de préparation des composés de formule (I) : 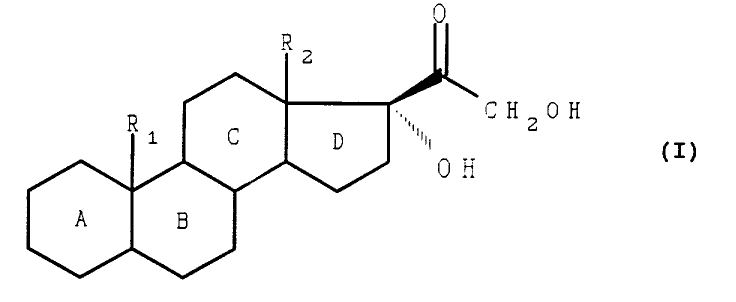 dans laquelle R₁ représente un atome d'hydrogène ou un radical alkyle renfermant de 1 à 4 atomes de carbone, R₂ représente un radical alkyle renfermant de 1 à 4 atomes de carbone, les noyaux A, B, C et D portent une ou plusieurs doubles liaisons et sont éventuellement substitués par une ou plusieurs fonctions hydroxyle ou cétone, le cas échéant protégées, par un ou plusieurs atomes d'halogène ou par un ou plusieurs radicaux alkyle ou alkyloxy renfermant de 1 à 4 atomes de carbone, ou par un ou plusieurs radicaux alkényle ou alkynyle renfermant de 2 à 4 atomes de carbone, caractérisé en ce que l'on traite un composé de formule (II) : 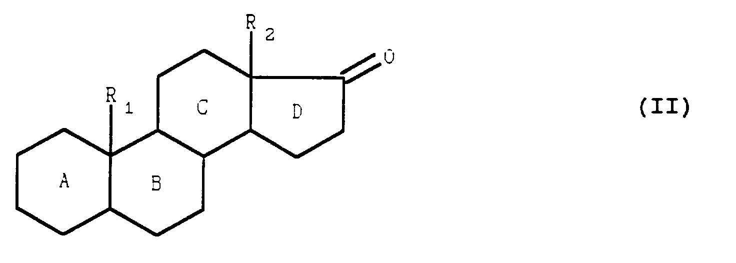 dans laquelle R₁, R₂, A, B, C et D sont définis comme précédemment, par un réactif de formule (R) : 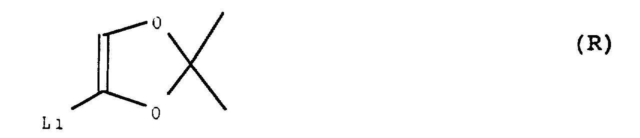 pour obtenir un composé de formule (III) : 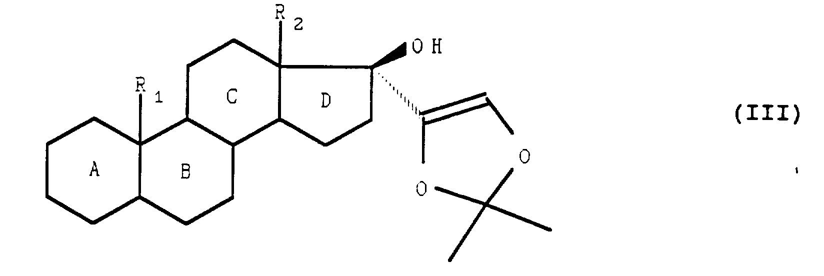 dans laquelle R₁, R₂, A, B, C et D sont définis comme précédemment, que l'on soumet à l'action d'un halogénure d'aryl sulfényle de formule (S) :
Ar-S-Hal (S)
dans laquelle Ar représente un radical phényle éventuellement substitué et Hal représente un atome d'halogène, en présence d'une base, pour obtenir intermédiairement un composé de formule (IV) : 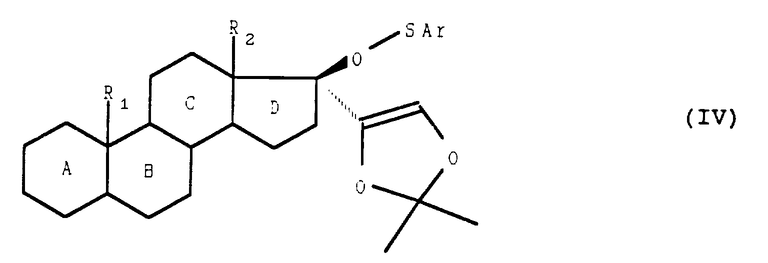 dans laquelle Ar est défini comme précédemment, que l'on réarrange, le cas échéant, en présence d'une base, en sulfoxyde de formule (V) : 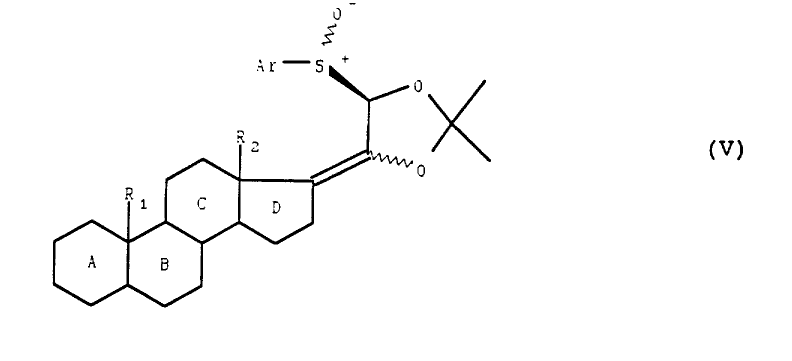 lequel se présente sous la forme d'un mélange de diastéréoisomères, de configuration E + Z, au niveau de la double liaison 17(20), sulfoxydes que l'on soumet à l'action d'un agent d'épimérisation, pour obtenir les sulfoxydes de formule (VI) : 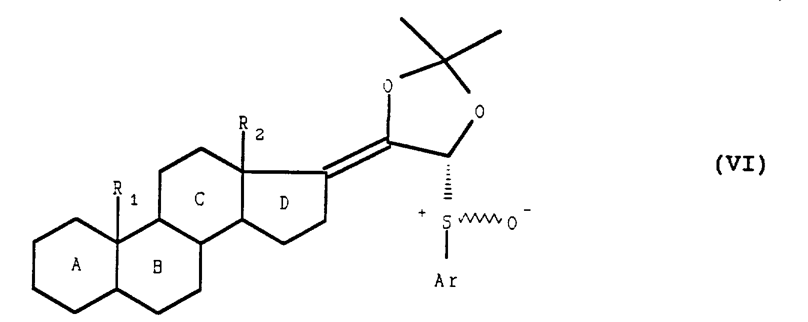 lesquels se présentent sous la forme d'un mélange de diastéréoisomères, de configuration (Z) au niveau de la double liaison 17(20), et qui sont en équilibre avec le sulfénate de formule (VII) : 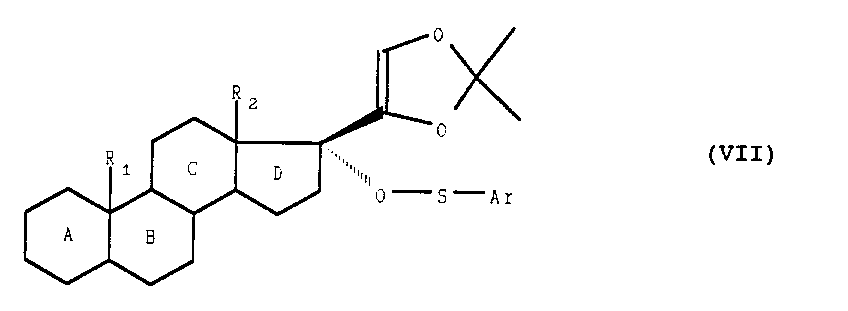 que l'on clive par un thiophile pour obtenir un composé de formule (VIII) : 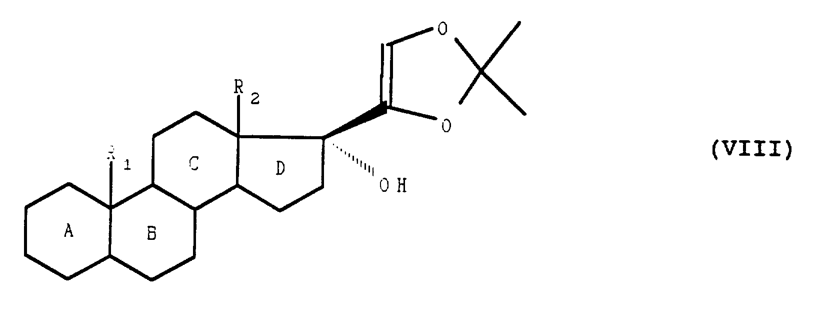 que l'on traite par un agent d'hydrolyse acide pour obtenir le composé de formule (I) attendu.

2. Procédé selon la revendication 1, caractérisé en ce que l'on utilise au départ un composé de formule (II) répondant à la formule (II') : 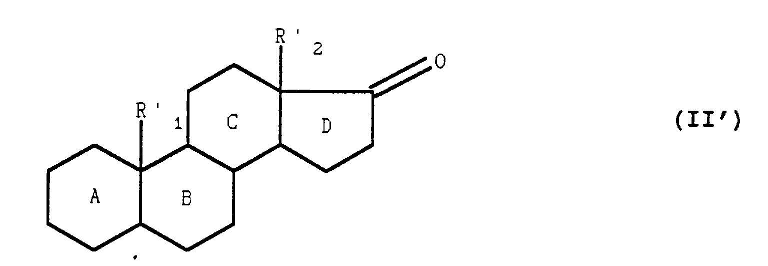 dans laquelle R'₁ représente un atome d'hydrogène ou un radical méthyle, R'₂ représente un radical méthyle ou éthyle, les noyaux A, B, C et D portent une ou plusieurs doubles liaisons en position 1(2), 3(4), 4(5) ou 9(11) ou 3(4) et 5(6) ou 4(5) et 6(7) ou 1(2) et 4(5) ou 1,3,5(10) ou 1(2), 4(5) et 6(7) et sont éventuellement substitués par une ou plusieurs fonctions hydroxyle en 3, 9 et/ou 11, par une ou deux fonctions cétone en 3 et/ou 11, la fonction cétone en 3 étant protégée, par un ou deux atomes de fluor, de chlore ou de brome en 6 et/ou 9α, par un ou plusieurs radicaux méthyle ou éthyle en 2, 6, 7 et/ou 16α ou 16β, par un ou deux radicaux méthoxy ou éthoxy en 3 et/ou 11β, par un radical vinyle ou allyle en 6 ou 11β ou par un radical éthynyle en 6 ou 11β.

3. Procédé selon la revendication 1 ou 2, caractérisé en ce que l'on utilise au départ un composé de formule (II) répondant à la formule (II'') : 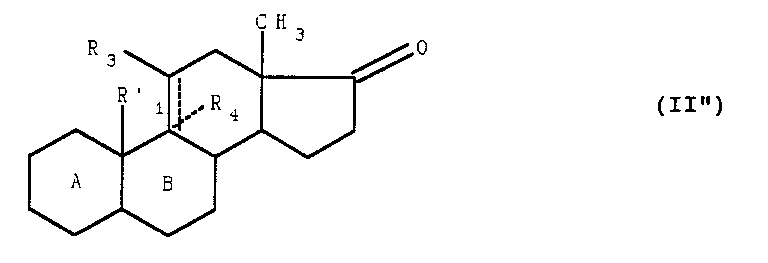 dans laquelle R'₁ est défini comme précédemment, le trait pointillé en 9(11) représente une éventuelle seconde liaison, R₃ représente un atome d'hydrogène, un radical oxo ou un radical hydroxy éventuellement protégé sous forme d'éther ou d'ester, R₄ représente un atome d'hydrogène, un radical hydroxy ou un atome de fluor, les cycles A et B représentent un reste : 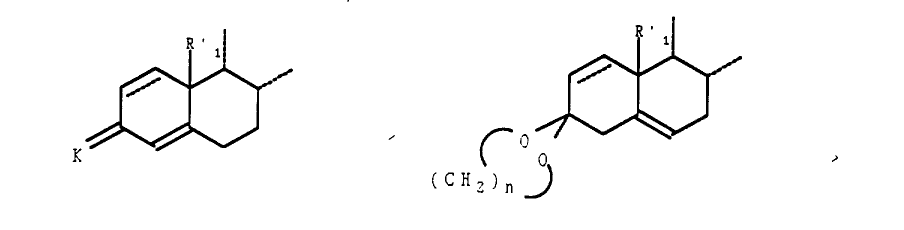 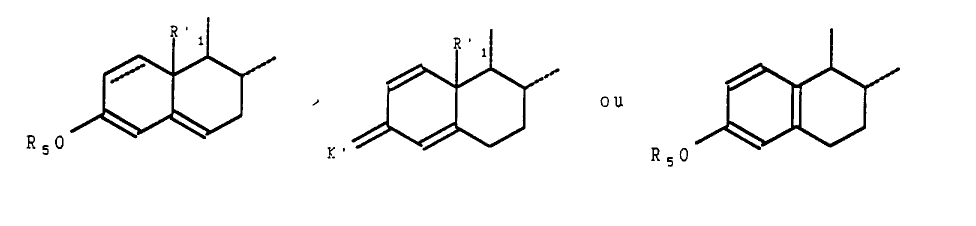 dans lequel le trait pointillé dans le cycle A représente une éventuelle seconde liaison, K représente un radical oxo protégé sous la forme : 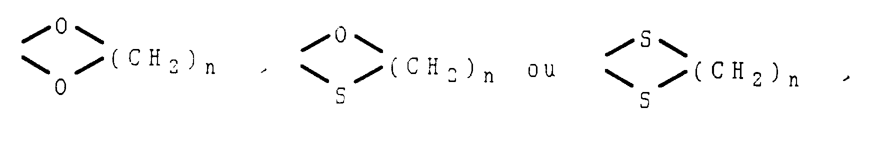 n étant égal à 2 ou 3, R₅ représente un reste éther ou ester, K' représente un radical oxo protégé sous la forme oxime, hydrazone ou semi-carbazone.

4. Procédé selon l'une quelconque des revendications 1 à 3, caractérisé en ce que l'agent de sulfénylation de formule (S) est choisi parmi le chlorure ou bromure de phényl, d'orthonitrophényle ou d'ortho-paradinitrophényl sulfényle.

5. Procédé selon l'une quelconque des revendications 1 à 4, caractérisé en ce que l'on effectue la réaction de sulfénylation en présence d'une base qui est une amine tertiaire choisie parmi la triéthylamine, la pyridine, la diméthylaminopyridine, le N-méthylimidazole ou le diazabicyclo-octane, ou un mélange d'amines tertiaires.

6. Procédé selon l'une quelconque des revendications 1 à 5, caractérisé en ce que l'on effectue le réarrangement thermiquement, en laissant remonter la température jusqu'à la température ambiante.

7. Procédé selon la revendication 6, caractérisé en ce que l'on ajoute au milieu réactionnel un excès de base telle que définie à la revendication 5.

8. Procédé selon l'une quelconque des revendications 1 à 7, caractérisé en ce que l'agent d'épimérisation est une base forte, choisie parmi les hydroxydes d'ammonium, les hydroxydes alcalins et les carbonates ou bicarbonates alcalins et en ce que l'on opère en milieu polaire, aqueux ou alcoolique.

9. Procédé selon la revendication 8, caractérisé en ce que la base est choisie parmi l'hydroxyde de N-benzyltriméthylammonium, l'hydroxyde de tétrabutylammonium, la soude, la potasse et la lithine.

10. Procédé selon la revendication 8, caractérisé en ce que l'on opère dans le méthanol et en présence d'un cosolvant choisi parmi le diméthylsulfoxyde et le diméthylformamide.

11. Procédé selon l'une quelconque des revendications 1 à 10, caractérisé en ce que l'on réalise le clivage du sulfénate par action de la base telle que définie à la revendication 8 ou 9, d'un trialkylphosphite, d'une amine secondaire ou d'un thiolate.

12. Procédé selon l'une quelconque des revendications 1 à 11, caractérisé en ce que l'on effectue l'hydrolyse du composé de formule (VIII) par action d'un acide minéral ou organique, soit en milieu homogène, soit préférentiellement en milieu biphasique, en présence d'eau et d'un cosolvant organique halogéné ou d'un ester ou d'un éther ou d'un solvant aromatique.

13. Procédé selon l'une quelconque des revendications 1 à 12, caractérisé en ce que l'on opère sans isoler intermédiairement les composés de formules (III) à (VII).

14. A titre de composés industriels nouveaux, les composés de formules (IV), (V), (VI), (VII) et (VIII) et les composés de formule (III) à l'exception du dérivé de la méthylestrone.

## Patentansprüche

1. Verfahren zur Herstellung der Verbindungen der Formel (I): 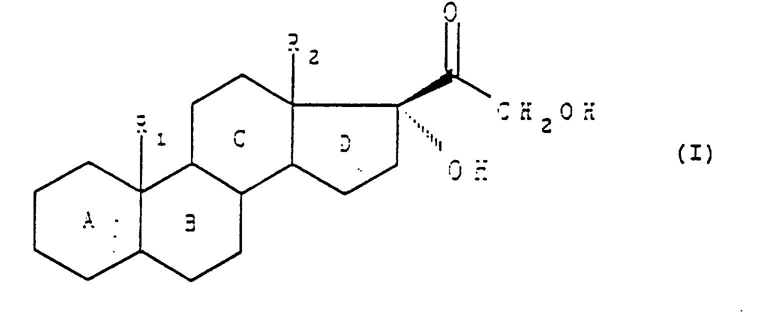 in der R₁ ein Wasserstoffatom oder einen Alkylrest mit 1 bis 4 Kohlenstoffatomen darstellt, R₂ einen Alkylrest mit 1 bis 4 Kohlenstoffatomen darstellt, die Ringe A, B, C und D eine oder mehrere Doppelbindungen tragen und gegebenenfalls mit einer oder mehreren, gegebenenfalls geschützten Hydroxy- oder Ketofunktionen, mit einem oder mehreren Halogenatomen oder mit einem oder mehreren Alkyl- oder Alkoxyresten mit 1 bis 4 Kohlenstoffatomen oder mit einem oder mehreren Alkenyl- oder Alkinylresten mit 2 bis 4 Kohlenstoffatomen substituiert sind, dadurch gekennzeichnet, daß man eine Verbindung der Formel (II): 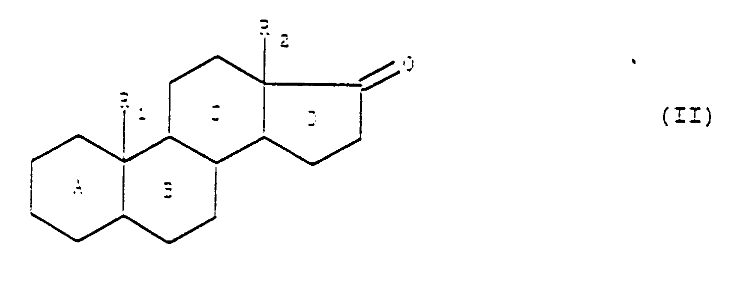 in der R₁, R₂, A, B, C und D wie zuvor definiert sind, mit einem Reagenz der Formel (R): 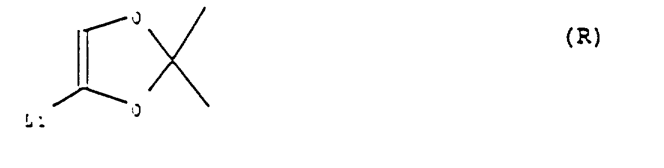 behandelt, um eine Verbindung der Formel (III): 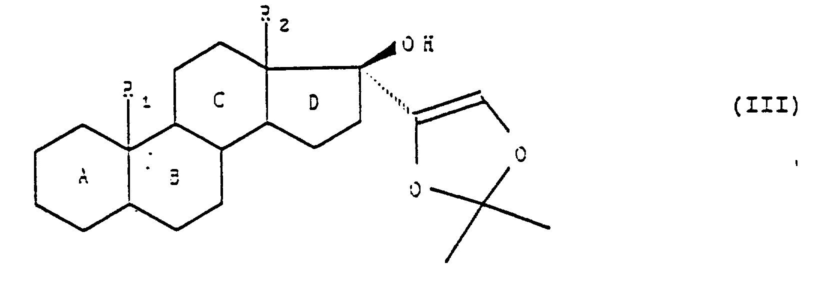 zu erhalten, in der R₁, R₂, A, B, C und D wie zuvor definiert sind, die man der Einwirkung eines Arylsulfenylhalogenids der Formel (S):
Ar-S-Hal (S)
in der Ar einen gegebenenfalls substituierten Phenylrest darstellt und Hal ein Halogenatom darstellt, in Gegenwart einer Base unterzieht, um intermediär eine Verbindung der Formel (IV): 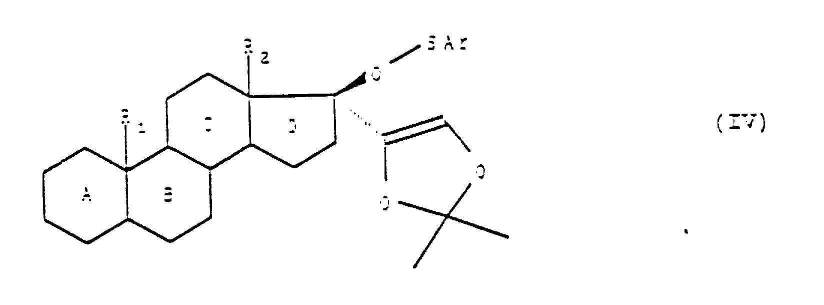 in der Ar wie zuvor definiert ist, zu erhalten, die man gegebenenfalls in Gegenwart einer Base in Sulfoxid der Formel (V): 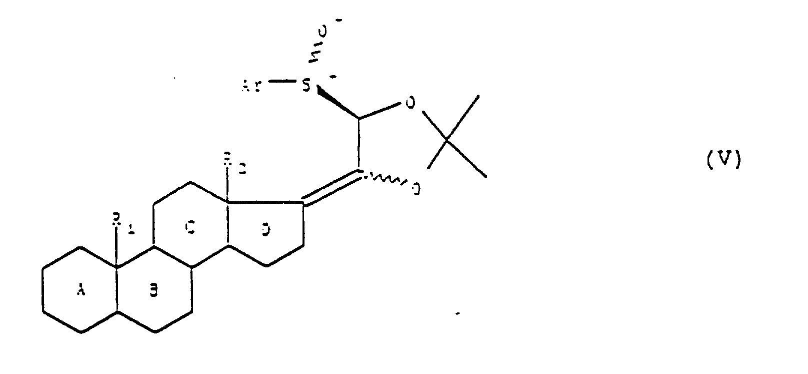 umlagert, das in der Form eines Diastereoisomerengemischs der Konfiguration E + Z an der Doppelbindung 17 (20) vorliegt, Sulfoxide, die man der Einwirkung eines Epimerisierungsmittels unterzieht, um die Sulfoxide der Formel (Vl): 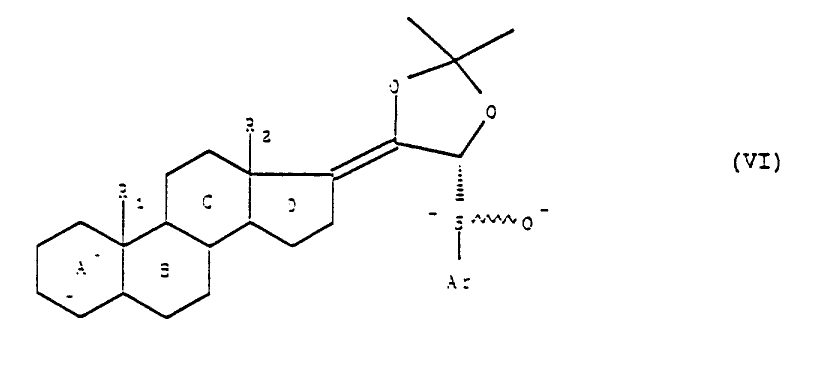 zu erhalten, die in der Form eines Diastereoisomerengemischs der Konfiguration (Z) an der Doppelbindung 17 (20) vorliegen und die im Gleichgewicht mit dem Sulfenat der Formel (VII): 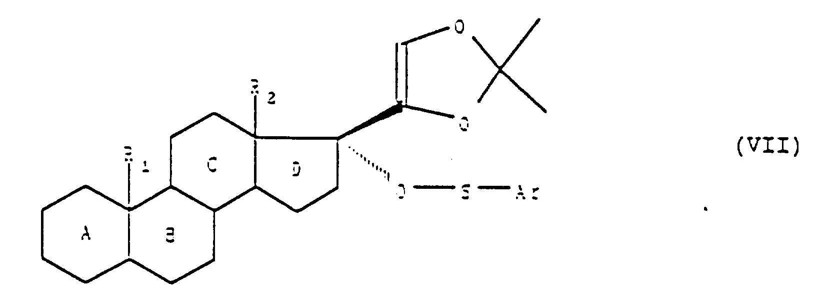 stehen, das man durch ein Thiophil spaltet, um eine Verbindung der Formel (VIII): 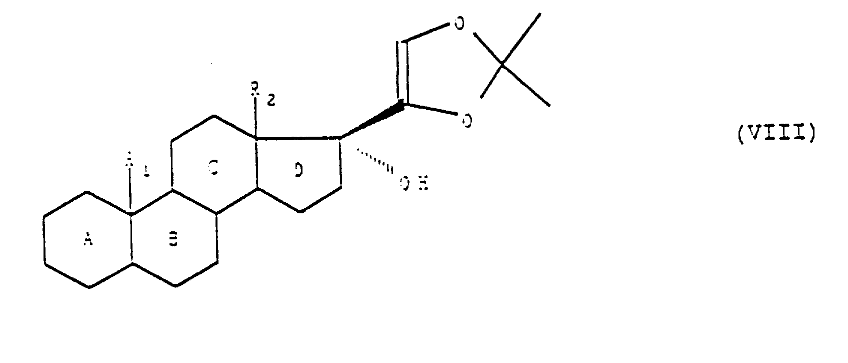 zu erhalten, die man mit einem sauren Hydrolysemittel behandelt, um die erwartete Verbindung der Formel (I) zu erhalten.

2. Verfahren gemäß Anspruch 1, dadurch gekennzeichnet, daß man zu Beginn eine Verbindung der Formel (II) verwendet, die der Formel (II'): 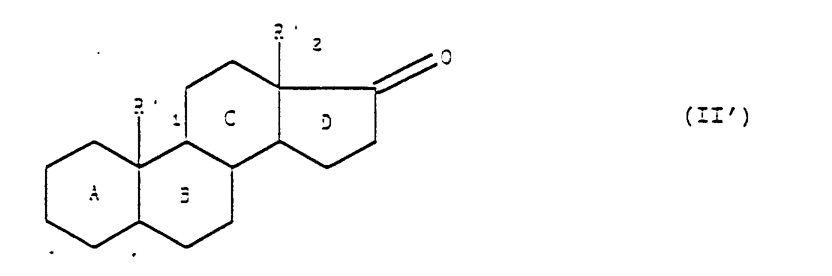 entspricht, in der R'₁ ein Wasserstoffatom oder einen Methylrest darstellt, R'₂ einen Methyl- oder Ethylrest darstellt, die Ringe A, B, C und D eine oder mehrere Doppelbindungen in Position 1 (2), 3 (4), 4 (5) oder 9 (11) oder 3 (4) und 5 (6) oder 4 (5) und 6 (7) oder 1 (2) und 4 (5) oder 1,3,5 (10) oder 1 (2), 4 (5) und 6 (7) tragen und gegebenenfalls mit einer oder mehreren Hydroxyfunktionen in 3, 9 und/oder 11, mit einer oder zwei Ketofunktionen in 3 und/oder 11, wobei die Ketofunktion in 3 geschützt ist, mit einem oder zwei Fluor-, Chlor- oder Bromatomen in 6 und/oder 9α, mit einem oder mehreren Methyl- oder Ethylresten in 2, 6, 7 und/oder 16α oder 16β, mit einem oder zwei Methoxy- oder Ethoxyresten in 3 und/oder 11 β, mit einem Vinyl- oder Allylrest in 6 oder 11β oder mit einem Ethinylrest in 6 oder 11β substituiert sind.

3. Verfahren gemäß Anspruch 1 oder 2, dadurch gekennzeichnet, daß man zu Beginn eine Verbindung der Formel (II) verwendet, die der Formel (ll"): 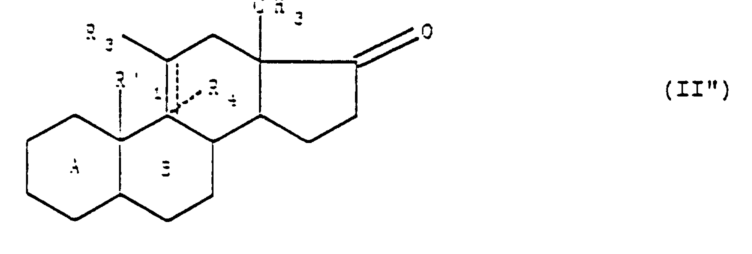 entspricht, in der R'₁ wie zuvor definiert ist, die punktierte Linie in 9 (11) eine etwaige zweite Bindung darstellt, R₃ ein Wasserstoffatom, einen Oxorest oder einen gegebenenfalls in Ether- oder Esterform geschützten Hydroxyrest darstellt, R₄ ein Wasserstoffatom, einen Hydroxyrest oder ein Fluoratom darstellt, die Ringe A und B einen Rest: 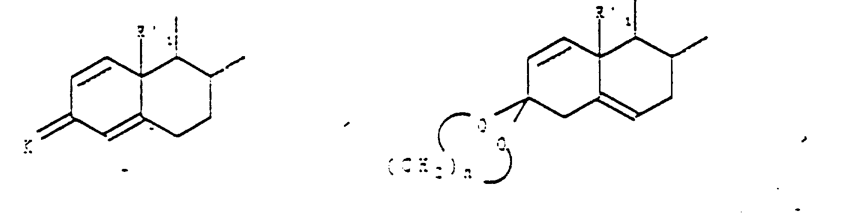 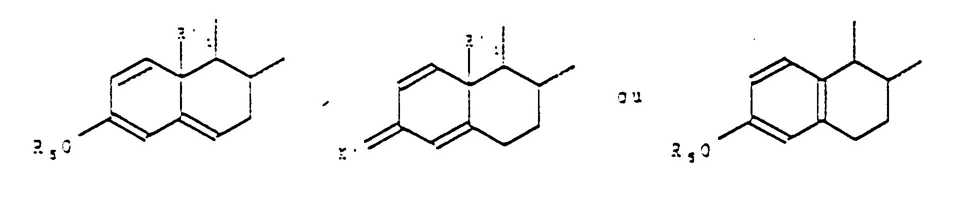 darstellen, in dem die punktierte Linie im Ring A eine etwaige zweite Bindung darstellt, K einen in der Form: 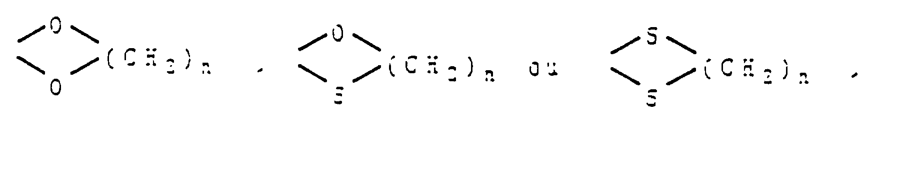 geschützten Oxorest darstellt, wobei n gleich 2 oder 3 ist, R5 einen Ether- oder Esterrest darstellt, K' einen in der Oxim-, Hydrazon- oder Semi-Carbazonform geschützten Oxorest darstellt.

4. Verfahren gemäß einem der Ansprüche 1 bis 3, dadurch gekennzeichnet, daß das Sulfenylierungsmittel der Formel (S) unter Phenyl-, o-Nitrophenyl- oder o,p-Dinitrophenylsulfenyl-chlorid oder -bromid ausgewählt ist.

5. Verfahren gemäß einem der Ansprüche 1 bis 4, dadurch gekennzeichnet, daß man die Sulfenylierungsreaktion in Gegenwart einer Base, die ein unter Triethylamin, Pyridin, Dimethylaminopyridin, N-Methylimidazol oder Diazabicyclooctan ausgewähltes tertiäres Amin oder ein Gemisch tertiärer Amine ist, ausführt.

6. Verfahren gemäß einem der Ansprüche 1 bis 5, dadurch gekennzeichnet, daß man die Umlagerung thermisch ausführt, indem man die Temperatur bis auf Raumtemperatur ansteigen läßt.

7. Verfahren gemäß Anspruch 6, dadurch gekennzeichnet, daß man dem Reaktionsmedium einen Überschuß an Base, wie in Anspruch 5 definiert, zugibt.

8. Verfahren gemäß einem der Ansprüche 1 bis 7, dadurch gekennzeichnet, daß das Epimerisierungsmittel eine starke Base ist, die unter den Ammoniumhydroxiden, den Alkalihydroxiden und den Alkalicarbonaten oder -bicarbonaten ausgewählt ist, und dadurch, daß man in wässrigem oder alkoholischem, polarem Medium arbeitet.

9. Verfahren gemäß Anspruch 8, dadurch gekennzeichnet, daß die Base unter N-Benzyltrimethylammoniumhydroxid, Tetrabutylammoniumhydroxid, Natriumhydroxid, Kaliumhydroxid und Lithiumhydroxid ausgewählt ist.

10. Verfahren gemäß Anspruch 8, dadurch gekennzeichnet, daß man in Methanol und in Gegenwart eines Hilfslösungsmittels, das unter Dimethylsulfoxid und Dimethylformamid ausgewählt ist, arbeitet.

11. Verfahren gemäß einem der Ansprüche 1 bis 10, dadurch gekennzeichnet, daß man die Spaltung des Sulfenats durch Einwirkung der wie in Anspruch 8 oder 9 definierten Base, eines Trialkylphosphits, eines sekundären Amins oder eines Thiolats ausführt.

12. Verfahren gemäß einem der Ansprüche 1 bis 11, dadurch gekennzeichnet, daß man die Hydrolyse der Verbindung der Formel (VIII) durch Einwirkung einer Mineralsäure oder organischen Säure entweder in homogenem Medium oder bevorzugt in zweiphasigem Medium in Gegenwart von Wasser und einem halogenierten organischen Hilfslösungsmittel oder einem Ester oder einem Ether oder einem aromatischen Lösungsmittel ausführt.

13. Verfahren gemäß einem der Ansprüche 1 bis 12, dadurch gekennzeichnet, daß man verfährt, ohne die Verbindungen der Formeln (III) bis (VII) intermediär zu isolieren.

14. Als neuartige industrielle Verbindungen die Verbindungen der Formeln (IV), (V), (Vl), (VII) und (VIII) und die Verbindungen der Formel (III) mit Ausnahme des Methylöstronderivats.

## Claims

1. Process for the preparation of compounds of the formula (I) : 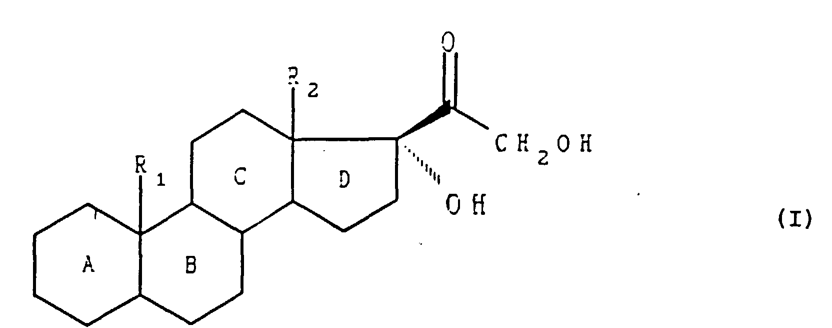 in which R₁ represents a hydrogen atom or an alkyl radical containing 1 to 4 carbon atoms, R₂ represents an alkyl radical containing 1 to 4 carbon atoms, the rings A, B, C and D carry one or more double bonds and are optionally substituted by one or more hydroxyl or ketone functions, which are protected if appropriate, by one or more halogen atoms or by one or more alkyl or alkyloxy radicals containing 1 to 4 carbon atoms, or by one or more alkenyl or alkinyl radicals containing 2 to 4 carbon atoms, characterized in that a compound of the formula (II): 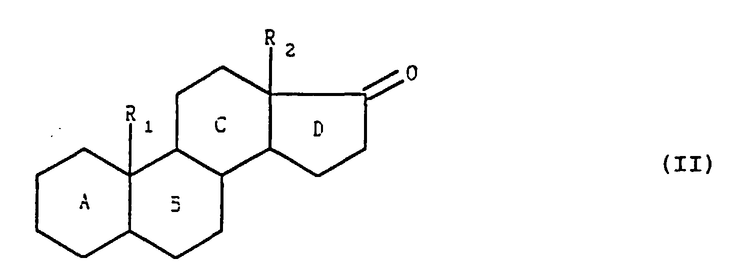 in which R₁, R₂, A, B, C and D are as defined above, is treated with a reagent of the formula (R): 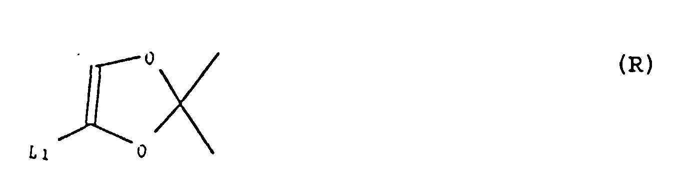 to give a compound of the formula (III): 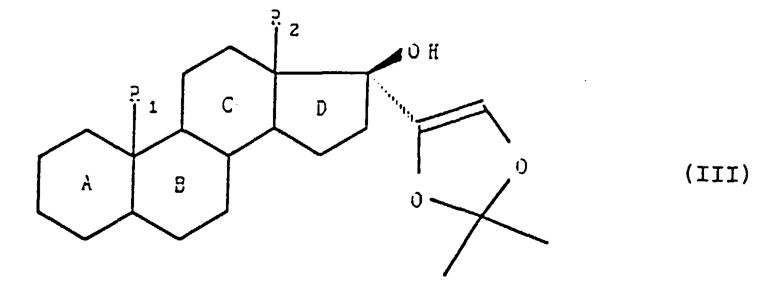 in which R₁, R₂, A, B, C and D are as defined above, which is subjected to the action of an arylsulphenyl halide of the formula (S):
Ar-S-Hal (S)
in which Ar represents an optionally substituted phenyl radical and Hal represents a halogen atom, in the presence of a base to give intermediately a compound of the formula (IV): 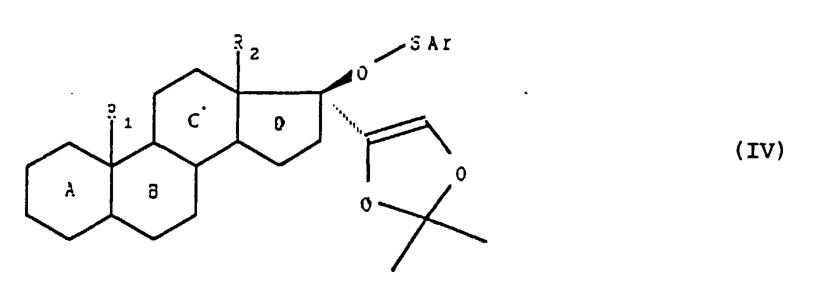 in which Ar is as defined above, which is rearranged, where appropriate, in the presence of a base into a sulphoxide of the formula (V): 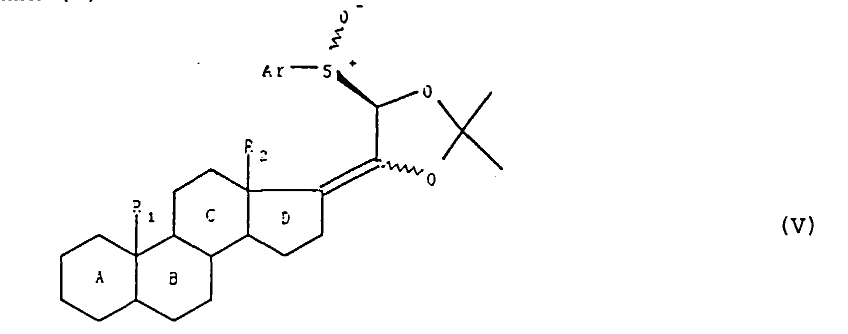 which is in the form of a mixture of diastereoisomers of the E + Z configuration on the 17(20) double bond, which sulphoxides are subjected to the action of an epimerizing agent to give the sulphoxides of the formula (VI): 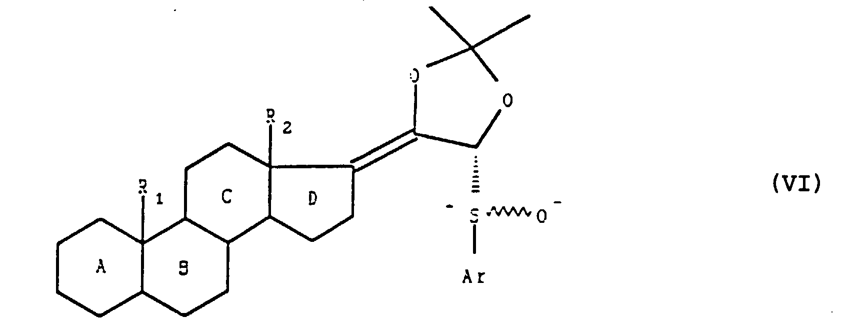 which are in the form of a mixture of diastereoisomers of the (Z) configuration on the 17(20) double bond, and which are in equilibrium with the sulphenate of the formula (VI): 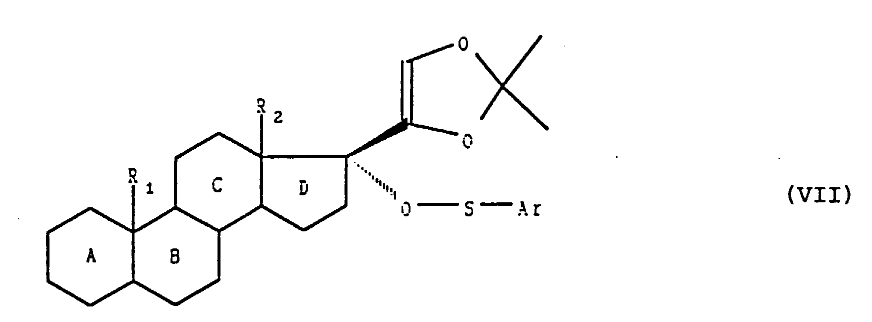 which is cleaved by a thiophile to give a compound of the formula (VIII): 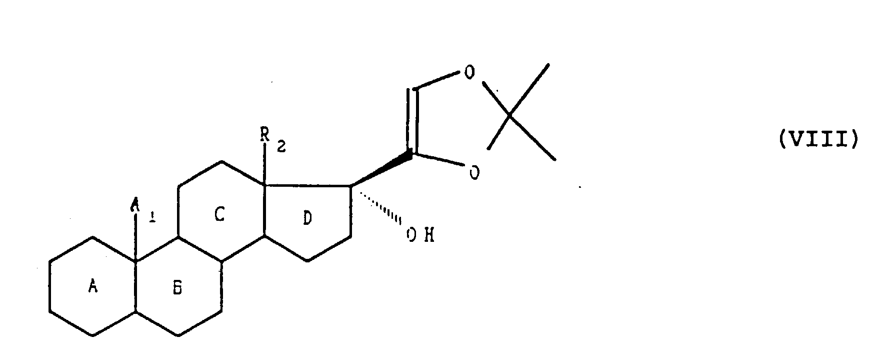 which is treated with an acid hydrolysis agent to give the expected compound of the formula (I).

2. Process according to claim 1, characterized in that a starting compound of the formula (II) corresponding to the formula (II'): 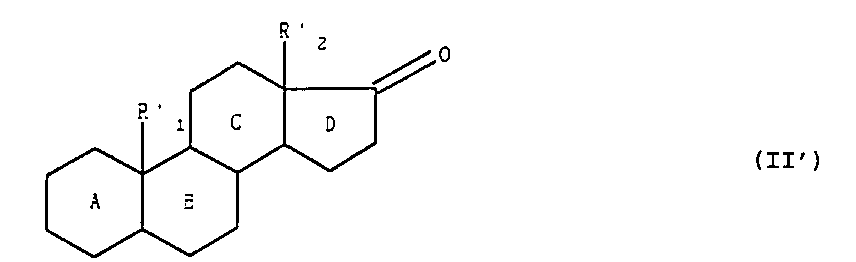 in which R'₁ represents a hydrogen atom or a methyl radical, R'₂ represents a methyl or ethyl radical, the rings A, B, C and D carry one or more double bonds in position 1(2), 3(4), 4(5) or 9(11) or 3(4) and 5(6) or 4(5) and 6(7) or 1(2) and 4(5) or 1,3,5(10) or 1(2), 4(5) and 6(7) and are optionally substituted by one or more hydroxyl functions in position 3, 9 and/or 11, by one or two ketone functions in position 3 and/or 11, the ketone function in position 3 being protected, by one or two fluorine, chlorine or bromine atoms in position 6 and/or 9α, by one or more methyl or ethyl radicals in position 2, 6, 7 and/or 16α or 16β, by one or two methoxy or ethoxy radicals in position 3 and/or 11β, by a vinyl or allyl radical in position 6 or 11β or by an ethynyl radical in position 6 or 11β, is used.

3. Process according to claim 1 or 2, characterized in that a starting compound of the formula (II) corresponding to the formula (II'): 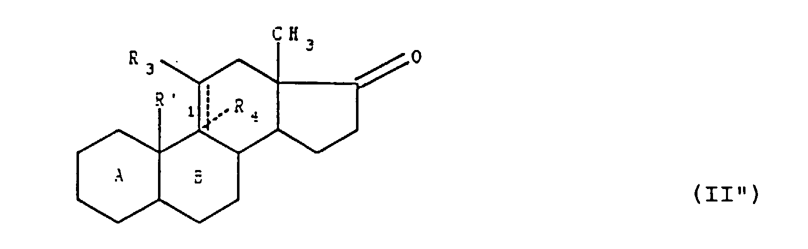 in which R'₁ is as defined above, the dotted line in position 9(11) represents an optional second bond, R₃ represents a hydrogen atom, an oxo radical or a hydroxyl radical optionally protected in the form of an ether or ester, R₄ represents a hydrogen atom, a hydroxyl radical or a fluorine atom, the rings A and B represent a residue: 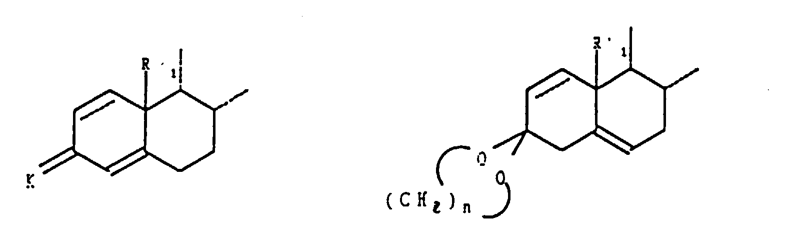 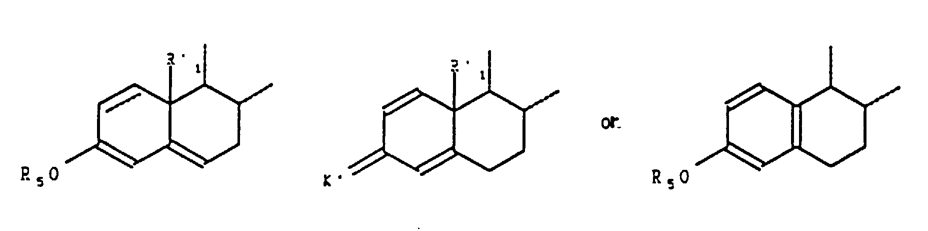 in which the dotted line in ring A represents an optional second bond, K represents an oxo radical protected in the form: 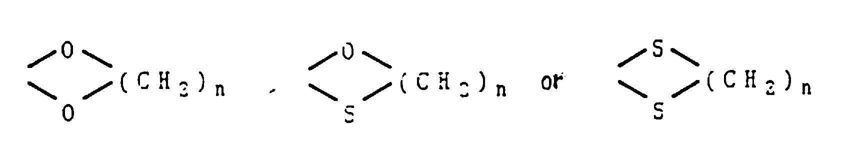 n being equal to 2 or 3, R₅ represents an ether or ester radical and K' represents an oxo radical protected in the form of an oxime, hydrazone or semi-carbazone, is used.

4. Process according to any one of claims 1 to 3, characterized in that the sulphenylating agent of the formula (S) is chosen from phenyl-, ortho-nitrophenyl- or ortho-paradinitrophenylsulphenyl chloride or bromide.

5. Process according to any one of claims 1 to 4, characterized in that the sulphenylation reaction is carried out in the presence of a base, which is, in particular, a tertiary amine chosen from triethylamine, pyridine, dimethylaminopyridine, N-methylimidazole or diazabicyclooctane, or a mixture of tertiary amines.

6. Process according to any one of claims 1 to 5, characterized in that the rearrangement is carried out by means of heat, by allowing the temperature to return to room temperature.

7. Process according to claim 6, characterized in that an excess of base as defined in claim 5 is added to the reaction mixture.

8. Process according to any one of claims 1 to 7, characterized in that the epimerizing agent is a strong base chosen from ammonium hydroxides, alkali metal hydroxides and alkali metal carbonates or bicarbonates, and in that the reaction is carried out in a polar, aqueous or alcoholic medium.

9. Process according to claim 8, characterized in that the base is chosen from N-benzyltrimethylammonium hydroxide, tetrabutylammonium hydroxide, sodium hydroxide, potassium hydroxide and lithium hydroxide.

10. Process according to claim 8, characterized in that it is carried out in methanol and in the presence of a cosolvent chosen from dimethylsulphoxide and dimethylformamide.

11. Process according to any one of claims 1 to 10, characterized in that the cleavage of the sulphenate is carried out by the action of the base as defined in claim 8 or 9, of a trialkyl phosphite, of a secondary amine or of a thiolate.

12. Process according to any one of claims 1 to 11, characterized in that the hydrolysis of the compound of the formula (VIII) is carried out by the action of a mineraal acid, either in a homogeneous medium or, preferably, in a biphase medium, in the presence of water and an organic halogenated cosolvent or an ester or an ether or an aromatic solvent.

13. Process according to any one of claims 1 to 12, characterized in that it is carried out without intermediate isolation of the compounds of the formulae (III) to (VII).

14. As new industrial compounds, the compounds of the formulae (IV), (V), (VI), (VII) and (VIII) and the compounds of the formula (III) with the exception of the methyloestrone derivative.
